# EUROPÄISCHE PATENTANMELDUNG

(11) **EP 2 626 047 A1**
(43) Veröffentlichungstag der Anmeldung: **14.08.2013**
(21) Anmeldenummer: 13154615.2
(22) Anmeldetag: 08.02.2013
(51) Int. Cl.: A61F 5/01, B25J 9/00

(54) **Orthese**

(30) Priorität: 09.02.2012 DE 102012002554
(71) Anmelder: Pohlig GmbH, 83278 Traunstein (DE)
(72) Erfinder: Pohlig, Kurt, 83278 Traunstein (DE); Schäfer, Michael, 83278 Traunstein (DE); Kienzle, Christian, 83064 Raubling (DE)
(74) Vertreter: Pfenning, Meinig & Partner GbR

(57) **Zusammenfassung**

Orthese (100) mit mindestens einem ersten Halteteil (2) und einem zweiten Halteteil (3), mindestens einem Orthesengelenk (4a, 4a'), welches das erste Halteteil mit dem zweiten Halteteil beweglich zueinander verbindet, und mindestens einem Gelenkantrieb (5a, 5b) zum Bewegen des Orthesengelenks, wobei das erste Halteteil geeignet ist zum Befestigen der Orthese an einem ersten Körperteil und das zweite Halteteil geeignet ist zum Führen eines mit dem ersten Körperteil durch ein Gelenk verbundenen zweiten Körperteils entlang vorbestimmter Freiheitsgrade bezüglich des ersten Körperteils, dadurch gekennzeichnet, dass die Orthese Befestigungselemente (12) aufweist, mittels derer der Gelenkantrieb jederzeit zur muskulär und/oder Eigenkraft geführten Bewegung der Halteteile zueinander von dem Orthesengelenk entkoppelbar ist und zur elektronisch gesteuerten Bewegung der Halteteile zueinander an das Orthesengelenk ankoppelbar ist.

## Beschreibung

Die Erfindung betrifft eine Orthese, insbesondere zur Korrektur von Fehlstellungen eines zwei Körperteile verbindenden Gelenks sowie zur Beübung des Gelenks.

Derartige Orthesen weisen üblicherweise zwei Schalenteile und ein die Schalenteile verbindendes Orthesengelenk auf. Die Schalenteile werden an den durch das Gelenk miteinander verbundenen Körperteilen befestigt und richten das Gelenk auf diese Weise derart aus, dass eine Bewegung des Gelenks nur entlang vorbestimmter Freiheitsgrade und in einem fest vorgegebenen Bewegungsbereich möglich ist.

Orthesen werden insbesondere nach einer Operation als auch zur konservativen Therapie eines Gelenks angewendet, um post-operative Gelenkeinsteifungen oder einen Rückfall in eine bereits durch Operation korrigierte Gelenkfehlstellung zu vermeiden.

Im Stand der Technik sind sowohl passive als auch aktive Orthesen bekannt. Passive Orthesen ermöglichen die selbstständige Bewegung eines Gelenks durch Muskelkraft innerhalb eines vorbestimmten Bewegungsbereichs und entlang vorbestimmter Freiheitsgrade.

Die Druckschrift DE 10 2008 012 996 A1 beschreibt eine aktive Orthese zur Korrektur von Fehlstellungen eines Gelenks. Die Orthese weist zwei Schalenteile zur Aufnahme zweier jeweils an das Gelenk angrenzender Körperteile auf. Dabei sind die Schalenteile durch ein Orthesengelenk derart miteinander verbunden, dass die Schalenteile mit den aufgenommenen Körperteilen von einer eventuell vorhandenen Gelenkfehlstellung in die Gelenksollstellung bringbar sind. Mit solchen Orthesen sollen Gelenkfehlstellungen dauerhaft zur Gelenksollstellung hin korrigiert werden, mit dem Ziel, dass die Gelenksollstellung schließlich auch ohne Orthese dauerhaft beibehalten wird. Mit der beschriebenen Orthese können außerdem Bewegungstherapien durchgeführt werden, ohne dass die Gefahr besteht, dass sich am Gelenk beteiligte Knochen in eine unerwünschte Fehlstellung bewegen. Dies wird dadurch erreicht, dass die zwei Schalenteile mit einer steuerbaren Bewegungseinrichtung verbunden sind, mit der die Schalenteile relativ zueinander bewegt werden können. Die Bewegungseinrichtung ist dabei fest mit den Schalenteilen verbunden und weist einen Motor zur fremdgesteuerten Bewegung des Orthesengelenks auf.

Die Druckschrift US 2005/0033208 A1 offenbart eine Therapie-Orthese, auch Quengel-Orthese genannt, zur Mobilisierung von versteiften menschlichen Gelenken oder Korrektur von Fehlstellungen von menschlichen Extremitäten. Die Quengel-Orthese wird dabei gelenkübergreifend an den jeweiligen Extremitäten so aufgenommen, dass das in einer krankhaften Beuge- oder Streckstellung fixierte Gelenk in seiner Bewegungseinschränkung korrigiert wird. Die Quengel-Orthese weist hierzu typischerweise zwei gegeneinander schwenkbare Orthesenteile auf, die mittels eines mechanischen Quengel-Zuges bzw. Quengel-Druckes zueinander bewegt werden, wodurch das Gelenk der in der Quengel-Orthese liegenden Extremität der jeweiligen Normalposition näher gebracht wird. Eine reproduzierbare und kontrollierbare Einstellung der Quengel-Kräfte, die auf das Gelenk wirken, ist bei derartigen Systemen jedoch meist schwierig. Die hier beschriebene Quengel-Orthese setzt hierzu anstelle eines mechanischen Systems ein über eine Steuereinheit pneumatisch gesteuertes Drucksteuersystem bzw. Druckregelsystem ein, um die Quengel-Kräfte vorzugeben. Die durch den Druck ausgeübte Quengel-Kraft der Quengel-Orthese ist damit exakt und reproduzierbar vorgebbar. Dabei erfolgt die Vorgabe der Quengel-Kräfte auch über längere Zeiträume genau entsprechend der vom Anwender wählbaren Vorgaben und kann zudem über entsprechende Anzeigen an einer Steuereinheit kontrolliert werden. Dadurch ist eine vollständige und genaue Therapiekontrolle während der gesamten Behandlungsdauer gewährleistet.

Nachteilig an den im Stand der Technik bekannten Orthesen ist, dass im Alltag und zur fremdgesteuerten Bewegungstherapie verschiedene Orthesen notwendig sind. Durch das Wechseln von der passiven Alltags-Orthese in die aktive Therapie-Orthese kann das Gelenk nicht in seinem individuell eingestellten, durch die Zwangsbedingungen der Alltags-Orthese vorgegebenen Bewegungsschema verbleiben. Desweiteren ist es auch nicht möglich, jederzeit und an jedem Ort zwischen einer passiven und einer aktiven Therapieeinheit zu wechseln, ohne eine zweite Orthese zur Verfügung zu haben.

Aufgabe der vorliegenden Erfindung ist es daher, eine Orthese bereitzustellen, mit der wahlweise eine aktive und eine passive Nutzung der Orthese ermöglicht wird, ohne beim Wechsel von der passiven in die aktive Nutzung oder umgekehrt das präzise voreingestellte Bewegungsschema des Gelenks zu verändern.

Diese Aufgabe wird durch die Orthese nach Anspruch 1, die Anordnung nach Anspruch 5, das Verfahren nach Anspruch 8 sowie die Verwendung nach Anspruch 10 gelöst. Vorteilhafte Weiterbildungen der erfindungsgemäßen Orthese sind in den abhängigen Ansprüchen 2 bis 4, der erfindungsgemäßen Anordnung in abhängigen Ansprüchen 5 bis 7 sowie des erfindungsgemäßen Verfahrens in dem abhängigen Anspruch 9 beschrieben.

Die vorliegende Erfindung ermöglicht die Durchführung von Bewegungstherapien für ein zwei Körperteile verbindendes Gelenk mit einer Orthese, die auch im Alltag getragen wird. Hierdurch kann der Patient die Bewegungstherapie mit der für ihn und auf das spezielle Gelenk eingestellte Alltags-Orthese durchführen. Dies ersetzt die unpräzisere Beübung des Gelenks mittels einer Therapie-Orthese, die für einen großen Patientenkreis verwendet wird und nur bedingt individuell und aufwendig immer wieder erneut einzustellen ist. Durch die Erfindung fällt ferner der Wechsel zwischen der passiven Alltags-Orthese und der Therapie-Orthese weg. Dies verhindert, dass die Fehlstellung des Gelenks durch zwei verschieden eingestellte Bewegungsschemata zweier unterschiedlicher Orthesen in unterschiedlicher Art und Weise korrigiert wird. Außerdem kann die Bewegungstherapie unabhängig vom Therapeuten durchgeführt werden.

Die erfindungsgemäße Orthese weist mindestens ein erstes Halteteil zur Befestigung an einem ersten Körperteil, beispielsweise einer ersten Gliedmaße, und ein zweites Halteteil zur Befestigung an einem zweiten Körperteil, beispielsweise einer mit der ersten Gliedmaße durch ein Gelenk verbundenen zweiten Gliedmaße, sowie mindestens ein Orthesengelenk auf, welches das erste Halteteil mit dem zweiten Halteteil beweglich zueinander verbindet. Ferner weist die erfindungsgemäße Orthese mindestens einen Gelenkantrieb zum Bewegen des Orthesengelenks bzw. der Halteteile zueinander auf. Das zweite Halteteil ist zum Führen eines zweiten Körperteils, welches mit dem ersten Körperteil durch ein körperliches Gelenk verbunden ist, entlang vorbestimmter Freiheitsgrade bezüglich des ersten Körperteils geeignet. Diese erlaubten Freiheitsgrade des zweiten Körperteils bezüglich des ersten Körperteils ergeben sich aus dem voreingestellten Bewegungsschema, welches durch die Zwangsbedingungen der dem Gelenk auferlegten Haltungskorrektur vorgegeben ist.

Der Gelenkantrieb kann entweder monolateral, also nur auf einer Seite, beispielsweise auf der linken oder der rechten Seite, der Orthese, oder bilateral, also auf beiden Seiten, also auf der linken und der rechten Seite, der Orthese am Orthesengelenk angeordnet sein.

Die erfindungsgemäße Orthese ist weiter gekennzeichnet durch Befestigungselemente. Mittels dieser Befestigungselemente ist der Gelenkantrieb jederzeit zur muskulär und/oder Eigenkraft geführten Bewegung der Halteteile relativ zueinander bzw. zur muskulär und/oder Eigenkraft geführten Bewegung des Orthesengelenks von dem Orthesengelenk entkoppelbar und zur elektronisch gesteuerten Bewegung der Halteteile relativ zueinander bzw. zur elektronisch gesteuerten Bewegung des Orthesengelenks an das Orthesengelenk ankoppelbar. Mit Eigenkraft ist hier die Kraft gemeint, welche die Muskeln und Bänder des Patienten auf dessen Körpergelenk ausüben.

Der Gelenkantrieb ist vorzugsweise jederzeit zur muskulär und/oder Eigenkraft geführten Bewegung der Halteteile relativ zueinander bzw. zur muskulär und/oder Eigenkraft geführten Bewegung des Orthesengelenks von der Orthese abnehmbar und zur elektronisch gesteuerten Bewegung der Halteteile zueinander bzw. zur elektronisch gesteuerten Bewegung des Orthesengelenks an die Orthese anbringbar. Es ist auch möglich, den Gelenkantrieb zur elektronisch gesteuerten Bewegung der Halteteile zueinander anstelle des Orthesengelenks anzubringen. In diesem Fall wird das Orthesengelenk durch eine Einheit aus einem Orthesengelenk und einem Gelenkantrieb ausgetauscht. Hierdurch wird eine flachere und/oder kompaktere Bauweise des Gelenkbereichs der Orthese, in welchem erstes und zweites Halteteil miteinander verbunden sind, bzw. der Einheit aus Orthesengelenk und Gelenkantrieb ermöglicht.

Alternativ ist es auch möglich, den Gelenkantrieb mit einer Kupplung auszustatten, wodurch der Gelenkantrieb vom Orthesengelenk auskuppelbar bzw. in das Orthesengelenk einkuppelbar ist. Dies ermöglicht wiederum einen schnellen Wechsel zwischen einer passiven und einer aktiven Therapieeinheit. Ferner muss der Antrieb nicht komplett abgenommen werden und ist somit jederzeit verfügbar. Desweiteren kann dadurch schnell in den später beschriebenen Teach-in-Modus gewechselt werden bzw. vom später beschriebenen Teach-in-Modus in den Beübungsmodus wieder zurückgewechselt werden.

Der Gelenkantrieb der Orthese weist vorteilhafterweise einen elektronisch gesteuerten Motor und/oder Aktuator als Gelenkantrieb auf. Hierdurch kann eine Bewegung der Orthese bzw. des Orthesengelenks oder der Halteteile zueinander ohne Aufwendung von Muskelkraft durch den Patienten erfolgen.

Für den elektronisch gesteuerten Gelenkantrieb können verschiedene Antriebe verwendet werden, beispielsweise ein Naben-, Schnecken- oder Kolbenantrieb, ein pneumatischer Muskel, ein Pneumatikzylinder oder eine Gewindespindel. Dabei ermöglicht ein Schneckengetriebe hohe Momente und langsame Drehzahlen. Eine Gewindespindel bzw. ein Spindelgetriebe führt Beuge- oder Streckbewegungen der Orthese bzw. der Halteteile zueinander durch, indem eine Spindel ein- oder ausfährt.

Handelt es sich bei dem Gelenkantrieb um einen Schneckenantrieb mit einem Motor und einer Schnecke, können der Motor und die Schneckenwelle derart angeordnet sein, dass der Motor und die Schneckenwelle des Schneckenantriebs eine gemeinsame Drehachse aufweisen oder derart, dass eine erste Drehachse des Motors des Schneckenantriebs und eine zweite Drehachse der Schneckenwelle des Schneckenantriebs parallel zueinander liegen. Im Fall, dass die Drehachse des Motors und die Drehachse der Schneckenwelle parallel zueinander liegen, wird ein geringerer Bauraum für den gesamten Antrieb möglich, wodurch die Antriebseinheit, die den Antrieb enthält, kleiner und/oder kompakter gebaut werden kann.

Der Gelenkantrieb kann ferner feste Anschläge aufweisen. Hierdurch wird ein absoluter Bewegungsbereich des Orthesengelenks definiert, der den natürlichen Bewegungsbereich des die Körperteile verbindenden Gelenks nicht überschreitet. Hierdurch wird wiederum die Verletzungsgefahr des Gelenks verringert bzw. Verletzungen verhindert. Zusätzlich zu diesen festen Anschlägen oder auch anstelle der festen Anschläge kann der Gelenkantrieb mit mechanisch und/oder elektronisch einstellbaren Anschlägen zur Beschränkung des Bewegungsbereichs des ersten und des zweiten Halteteils zueinander ausgestattet sein. Die elektronisch einstellbaren Anschläge werden lediglich von einer Steuerungssoftware überwacht. Sollten diese elektronisch einstellbaren Anschläge ausfallen, beispielsweise durch einen Fehler in der Steuerungssoftware, verhindert das Vorhandensein von mechanisch einstellbaren Anschlägen eine Bewegung der Halteteile zueinander über den eingeschränkten Bewegungsbereich oder über einen maximal ohne Gesundheitsschäden zulässigen Bewegungsbereich hinaus.

Das erste und das zweite Halteteil der Orthese können ferner parallele erste und zweite Schienen aufweisen, welche in Längsrichtung der Halteteile, jeweils zueinander beabstandet angeordnet sind. Während der Verwendung der Orthese befinden sich die erste und die zweite Schiene des ersten Halteteils also an gegenüberliegenden Seiten, beispielsweise links und rechts, des ersten Körperteils und in etwa senkrecht zur Drehachse des Orthesengelenks. Gleichzeitig befinden sich die erste und die zweite Schiene des zweiten Halteteils an gegenüberliegenden Seiten, beispielsweise links und rechts, des zweiten Körperteils und ebenfalls in etwa senkrecht zur Drehachse des Orthesengelenks. Ferner sind jeweils die ersten Schienen miteinander und die zweiten Schienen miteinander über das Orthesengelenk zueinander beweglich verbunden. An der ersten Schiene des ersten Halteteils sowie an der ersten Schiene des zweiten Halteteils und/oder an der zweiten Schiene des ersten Halteteils sowie an der zweiten Schiene des zweiten Halteteils können Befestigungselemente zum Abnehmen bzw. Anbringen des Gelenkantriebs angeordnet sein. Diese Art der Anordnung der Befestigungselemente an den ersten und zweiten Schienen ist vorteilhaft bei der Verwendung eines Kolbenantriebs, eines pneumatischen Muskels, eines Pneumatik-Zylinders oder einer Gewindespindel.

Alternativ können die Befestigungselemente zum Abnehmen bzw. Anbringen des Gelenkantriebs auch an einem Drehpunkt und/oder an einer Drehachse des Orthesengelenks oder benachbart zu einer Drehachse und/oder einem Drehpunkt des Orthesengelenks angeordnet sein. Diese Art der Anordnung der Befestigungselemente ist besonders vorteilhaft bei der Verwendung eines Nabenantriebs oder eines Schneckenantriebs, da diese Antriebe direkt an der Drehachse bzw. an dem Drehpunkt des Orthesengelenks angreifen.

Die Befestigungselemente können vorteilhafterweise derart ausgebildet sein, dass der Gelenkantrieb werkzeugfrei abnehmbar bzw. anbringbar ist. Hierdurch kann die Orthese in sehr kurzer Zeit von einer passiven Alltags-Orthese zu einer aktiven elektronisch gesteuerten Therapie-Orthese umgewandelt und umfunktioniert werden.

Besonders vorteilhaft ist es, wenn die Befestigungselemente zum Abnehmen bzw. Anbringen des Gelenkantriebs Bestandteile eines Klemm-Aufnahme-Systems, eines Magnetstecksystems, eines Kugelkopfsystems oder eines Bajonett-Verschlusses sind. Für den Fall, dass die Befestigungselemente Bestandteile eines Klemm-Aufnahme-Systems sind, weisen die Befestigungselemente Hinterschneidungen und der Gelenkantrieb Klemmelemente auf, so dass die Klemmelemente mit den Befestigungselementen verklemmbar sind.

Für den Fall, dass die Befestigungselemente Bestandteile eines Magnetstecksystems sind, weisen die Befestigungselemente ringförmige, magnetische Aufnahmen und der Gelenkantrieb zylindrische, magnetische Stifte auf, so dass die Stifte in die Aufnahmen einsteckbar sind.

Für den Fall, dass die Befestigungselemente Bestandteile eines Kugelkopfsystems sind, weisen die Befestigungselemente kugelförmige Köpfe und der Gelenkantrieb pfannenförmige Klemmelemente mit Hinterschneidungen auf, so dass die Köpfe in die pfannenförmigen Klemmelemente einrastbar sind.

Die Orthese kann ferner derart ausgebildet sein, dass sie sich für ein körperliches Gelenk, welches zwei Körperteile miteinander verbindet, eignet, beispielsweise für ein Scharnier-, Kugel-, Hüft-, Knie-, Sprung- oder Ellbogengelenk.

Desweiteren ist es auch möglich, dass die Orthese ein drittes und/oder gegebenenfalls auch ein viertes Halteteil aufweist, wobei das dritte Halteteil mit dem ersten oder mit dem zweiten Halteteil über ein weiteres Orthesengelenk beweglich zum ersten oder zum zweiten Halteteil verbunden ist. Ist auch ein viertes Halteteil vorhanden, so ist dieses mit dem ersten, dem zweiten oder dem dritten Halteteil über ein weiteres Orthesengelenk beweglich zum ersten, zum zweiten oder zum dritten Halteteil verbunden. Dies ermöglicht die Korrektur von Fehlstellungen von mehreren Gelenken, welche die an das jeweilige Gelenk angrenzenden Körperteile miteinander verbinden.

Eine weitere mögliche Ausführungsform der Orthese ist dadurch gegeben, dass das zweite Halteteil eine Ringfassung, z.B. zur Aufnahme eines Fußes, aufweist. Eine solche Ringfassung bietet dem Fuß einen stärkeren Halt für eine noch gezieltere Korrektur der Fußstellung und/oder der Sprunggelenksstellung.

Weiterhin kann die erfindungsgemäße Orthese dadurch gekennzeichnet sein, dass der Gelenkantrieb einen Motor und/oder einen Aktuator aufweist.

Weiterhin kann die erfindungsgemäße Orthese dadurch gekennzeichnet sein, dass der Gelenkantrieb ein Naben-, Schnecken- oder Kolbenantrieb, ein pneumatischer Muskel, ein Pneumatikzylinder oder eine Gewindespindel ist.

Weiterhin kann die erfindungsgemäße Orthese dadurch gekennzeichnet sein, dass der Gelenkantrieb ein Schneckenantrieb mit einem Motor und einer Schnecke ist, wobei der Motor und die Schnecke des Schneckenantriebs eine gemeinsame Drehachse aufweisen oder wobei eine erste Drehachse des Motors des Schneckenantriebs und eine zweite Drehachse der Schnecke des Schneckenantriebs parallel zueinander angeordnet sind.

Weiterhin kann die erfindungsgemäße Orthese dadurch gekennzeichnet sein, dass der Gelenkantrieb feste Anschläge und/oder mechanisch einstellbare und/oder elektrisch einstellbare Anschläge zur Beschränkung des Bewegungsbereichs des ersten und zweiten Halteteils zueinander aufweist.

Weiterhin kann die erfindungsgemäße Orthese dadurch gekennzeichnet sein, dass das erste und das zweite Halteteil in Längsrichtung jeweils zueinander beabstandet angeordnete, parallele erste und zweite Schienen aufweisen, wobei jeweils die ersten Schienen und die zweiten Schienen über das Orthesengelenk beweglich zueinander verbunden sind.

Weiterhin kann die erfindungsgemäße Orthese dadurch gekennzeichnet sein, dass die Befestigungselemente zum Abnehmen und Anbringen des Gelenkantriebs an einem Drehpunkt und/oder an einer Drehachse des Orthesengelenks oder benachbart zu einem Drehpunkt und/oder einer Drehachse des Orthesengelenks angeordnet sind.

Weiterhin kann die erfindungsgemäße Orthese dadurch gekennzeichnet sein, dass die Befestigungselemente zum Abnehmen und Anbringen des Gelenkantriebs an der ersten Schiene des ersten Halteteils sowie an der ersten Schiene des zweiten Halteteils und/oder an der zweiten Schiene des ersten Halteteils sowie an der zweiten Schiene des ersten Halteteils angeordnet sind.

Weiterhin kann die erfindungsgemäße Orthese dadurch gekennzeichnet sein, dass die Befestigungselemente derart ausgebildet sind, dass der Gelenkantrieb werkzeugfrei abnehmbar und anbringbar ist.

Weiterhin kann die erfindungsgemäße Orthese dadurch gekennzeichnet sein, dass die Befestigungselemente zum Abnehmen und Anbringen des Gelenkantriebs Bestandteile eines Klemm-Aufnahme-Systems, eines Magnetstecksystems, eines Kugelkopfsystems oder eines Bajonett-Verschlusses sind.

Weiterhin kann die erfindungsgemäße Orthese dadurch gekennzeichnet sein, dass die Befestigungselemente Bestandteile des Klemm-Aufnahme-Systems sind, wobei die Befestigungselemente Hinterschneidungen aufweisen und der Gelenkantrieb Klemmelemente aufweist, so dass die Klemmelemente mit den Befestigungselementen verklemmbar sind.

Weiterhin kann die erfindungsgemäße Orthese dadurch gekennzeichnet sein, dass die Befestigungselemente Bestandteile des Magnetstecksystems sind, wobei die Befestigungselemente ringförmige, magnetische Aufnahmen aufweisen und der Gelenkantrieb zylindrische, magnetische Stifte aufweist, so dass die Stifte in die Aufnahmen einsteckbar sind.

Weiterhin kann die erfindungsgemäße Orthese dadurch gekennzeichnet sein, dass die Befestigungselemente Bestandteile des Kugelkopfsystems sind, wobei die Befestigungselemente kugelförmige Köpfe aufweisen und der Gelenkantrieb pfannenförmige Klemmelemente mit Hinterschneidungen aufweist, so dass die Köpfe in die pfannenförmigen Klemmelemente einrastbar sind.

Weiterhin kann die erfindungsgemäße Orthese dadurch gekennzeichnet sein, dass das die Körperteile verbindende Gelenk ein Scharnier-, Kugel-, Hüft , Knie-, Sprung- oder Ellbogengelenk ist.

Weiterhin kann die erfindungsgemäße Orthese dadurch gekennzeichnet sein, dass die Orthese ein drittes und/oder gegebenenfalls ein viertes Halteteil aufweist, wobei das dritte Halteteil mit dem ersten oder mit dem zweiten Halteteil über ein weiteres Orthesengelenk beweglich zum ersten oder/und zum zweiten Halteteil verbunden ist und das vierte Halteteil mit dem ersten, zweiten oder dritten Halteteil über ein weiteres Orthesengelenk beweglich zum ersten, zweiten, und/oder dritten Halteteil verbunden ist.

Weiterhin kann die erfindungsgemäße Orthese dadurch gekennzeichnet sein, dass das zweite Halteteil eine Ringfassung, z.B. zur Aufnahme eines Fußes, aufweist.

Die hier beschriebene Orthese kann ferner ein Teil einer Anordnung zur therapeutischen Beübung eines ersten und eines zweiten Körperteils und/oder eines die Körperteile verbindenden Gelenks sein. Die Anordnung weist außerdem einen Sensor zum Messen und/oder Überwachen einer Stellung und/oder einer Drehgeschwindigkeit des Orthesengelenks zu verschiedenen Zeitpunkten auf. Der Sensor ist vorteilhafterweise weiter geeignet zum Messen einer Mittelstellung des Orthesengelenks bzw. der Halteteile zueinander, einer Endstellung des Orthesengelenks bzw. der Halteteile zueinander, eines Beschleunigungsprofils und/oder eines Drehmoments des Orthesengelenks.

Ferner weist die Anordnung eine Steuereinheit auf, die ein Speicherelement zum Speichern von Therapiedaten, insbesondere von Messwerten des genannten Sensors, welche hier alternativ auch Sensorwerte genannt werden, eines Steuerungsablaufs und/oder von Bewegungen des Orthesengelenks bzw. der Halteteile zueinander während eines Trainingsablaufs, einen Mikroprozessor und ein Bedienelement zum Programmieren und Ausführen eines Bewegungsprogramms und ein Steuerelement zum Steuern des Gelenkantriebs beinhaltet. Die Anordnung ist außerdem mit einer Stromversorgung ausgestattet.
Vorzugsweise ist die Steuereinheit derart aufgebaut, dass das Speicherelement und/oder der Mikroprozessor in dem Bedienelement enthalten ist.

Die Steuereinheit zur therapeutischen Beübung des die Körperteile verbindenden Gelenks oder des ersten und zweiten Körperteils kann programmierbar eingerichtet sein. Dadurch kann vor einer aktiven Therapieeinheit mit der Orthese in die Steuereinheit ein Trainingsablauf einprogrammiert werden, nach welchem die Steuereinheit die Orthese während des Trainings steuert.

Insbesondere kann es vorteilhaft sein, die Steuereinheit mit einer Timer-Funktion zum automatischen Starten und Stoppen der therapeutischen Beübung auszustatten. Mittels einer derartigen Timer-Funktion lassen sich beispielsweise ein Start-Zeitpunkt und/oder die Dauer der Beübung und/oder ein Endzeitpunkt der Beübung programmieren.

Vorteilhafterweise weist die Steuereinheit einen Netzwerkanschluss zum Herstellen einer Verbindung zu einem drahtlosen Netzwerk auf. Ein drahtloses Netzwerk ist beispielsweise ein lokales Drahtlosnetzwerk (WLAN), ein Mobilfunknetz oder eine BluetoothVerbindung. Die dazugehörigen Netzwerkanschlüsse sind beispielsweise eine WLAN-Netzwerkkarte, eine Mobilfunkschnittstelle oder ein Bluetooth-Sender/Empfänger. Über ein drahtloses Netzwerk können in der Speichereinheit speicherbare Daten mittels eines Mikroprozessor-gesteuerten Geräts, insbesondere mittels eines tragbaren Mikroprozessor-gesteuerten Geräts abgerufen, ausgelesen oder übertragen werden. Als Mikroprozessor-gesteuerte Geräte eignen sich hierzu je nach Art des verfügbaren drahtlosen Netzwerks insbesondere ein Netbook, ein Laptop, ein Mobiltelefon, beispielsweise ein Smartphone. Das Abrufen, Auslesen und Übertragen der in der Speichereinheit gespeicherten Daten über ein drahtloses Netzwerk ermöglicht eine Dokumentation der Therapie, eine Qualitätssicherung der Therapie bzw. der Orthese oder eine Wartung oder Änderung der Orthese, insbesondere auch aus der Ferne. Des Weiteren können Trainingsprogramme über ein drahtloses Netzwerk, insbesondere aus der Ferne, an die Steuereinheit, bzw. an das Speicherelement der Steuereinheit, gesendet werden. Die Steuereinheit kann vorzugsweise auch direkt über das drahtlose Netzwerk programmiert werden. Dies ermöglicht dem Therapeuten die Therapieeinheit für die Bewegungstherapie, insbesondere aus der Ferne, vorzugeben, anzupassen und zu kontrollieren.

Alternativ kann die Steuereinheit zum Herstellen einer Verbindung zu einem drahtgebundenen Netzwerk ausgebildet sein. Ein solches drahtgebundenes Netzwerk ist z.B. ein lokales Netzwerk (LAN) oder das Internet. Zum Herstellen einer solchen drahtgebundenen Netzwerkverbindung kann die Steuereinheit einen Netzwerkanschluss, z.B. eine LAN-Netzwerkkarte, aufweisen, wodurch in der Speichereinheit speicherbare Daten über das drahtgebundene Netzwerk ebenfalls mittels eines Mikroprozessor-gesteuerten Geräts, insbesondere mittels eines tragbaren Mikroprozessor-gesteuerten Geräts, wie z.B. einem Netbook oder einem Laptop, abgerufen oder ausgelesen werden können. Des Weiteren können Trainingsprogramme über ein drahtgebundenes Netzwerk an die Steuereinheit, bzw. an das Speicherelement der Steuereinheit, gesendet werden. Die Steuereinheit kann vorzugsweise auch direkt über das drahtlose Netzwerk programmiert werden. Dies ermöglicht dem Therapeuten die Therapieeinheit für die Bewegungstherapie, insbesondere aus der Ferne, vorzugeben, anzupassen und zu kontrollieren.

Das Speicherelement kann eine Festplatte, ein Halbleiterlaufwerk, beispielsweise ein Flash-Speicher, oder eine Kombination dieser Speicherarten sein. Die Verwendung eines Halbleiterlaufwerks bzw. eines Flash-Speichers ist besonders vorteilhaft, da mit diesen die Datenübertragung schnell durchgeführt werden kann.

Ferner kann die Steuereinheit über einen Anschluss und/oder eine Schnittstelle für ein externes Speichermedium, z.B. für einen USB-Stick, eine Chip-Karte, eine Speicherkarte oder eine externe Festplatte, verfügen. Auf einem derartigen externen Speichermedium kann beispielsweise ein Therapie-Programm gespeichert und durch Anschließen des Speichermediums an die Steuereinheit übertragen werden. Ferner können Therapie-Daten, die während der Therapie abgefragt werden und den Therapiefortschritt dokumentieren, beispielsweise auf einer Chip-Karte gespeichert werden. Diese kann dann später mittels eines Lesegerätes vom Therapeuten ausgelesen werden.

Der in der Anordnung enthaltene Sensor kann ein Drehpotentiometer, ein Folienpotentiometer, ein integriertes Absolut-Potentiometer und/oder einen Hallsensor aufweisen.

Die Stromversorgung der Anordnung erfolgt vorteilhafterweise über einen Akkumulator oder über das Stromnetz. Eine Stromversorgung mittels eines Akkumulators ermöglicht die Verwendung eines elektronisch gesteuerten Gelenkantriebs an jedem Ort, ohne auf eine Stromnetzanbindung angewiesen zu sein.

Weiterhin kann die erfindungsgemäße Anordnung dadurch gekennzeichnet sein, dass das Speicherelement eine Festplatte, ein Festkörperlaufwerk und/oder ein Flash-Speicher ist.

Weiterhin kann die erfindungsgemäße Anordnung dadurch gekennzeichnet sein, dass die Steuereinheit über einen Anschluss und/oder eine Schnittstelle für ein externes Speichermedium, z.B. USB-Stick, Chip-Karte, Speicherkarte, externe Festplatte, verfügt.

Weiterhin kann die erfindungsgemäße Anordnung dadurch gekennzeichnet sein, dass die Steuereinheit zur therapeutischen Beübung des die Körperteile verbindenden Gelenks und/oder des ersten und zweiten Körperteils programmierbar ist und/oder eine Timer-Funktion zum automatischen Starten und/oder Stoppen der therapeutischen Beübung beinhaltet.

Weiterhin kann die erfindungsgemäße Anordnung dadurch gekennzeichnet sein, dass der Sensor ein Drehpotentiometer, ein Folienpotentiometer, ein integriertes Absolut-Potentiometer und/oder einen Hallsensor aufweist.

Weiterhin kann die erfindungsgemäße Anordnung dadurch gekennzeichnet sein, dass die Stromversorgung einen Akkumulator aufweist.

Die hier beschriebene Anordnung mit der oben beschriebenen Orthese kann des Weiteren in einem Verfahren zur therapeutischen Beübung eines Gelenks oder eines ersten Körperteils und eines mit dem ersten Körperteil durch ein Gelenk verbundenen zweiten Körperteils verwendet werden. Bei diesem Verfahren wird die Orthese mittels eines ersten Halteteils am ersten Körperteil befestigt, und das zweite Körperteil mittels des zweiten Halteteils entlang vorbestimmter Freiheitsgrade geführt.

Das Verfahren kann ferner dahingehend weitergebildet sein, dass mittels des Bedienelements und des Mikroprozessors eine Bewegungsvorlage programmiert werden kann.

Das Verfahren kann einen Teach-In-Modus zur Aufnahme von Sensorwerten mittels des Sensors beinhalten. In dem Teach-In-Modus wird der Gelenkantrieb von dem Orthesengelenk entkoppelt.

Der Teach-In-Modus des Verfahrens kann weiter beinhalten, dass die Halteteile zueinander bewegt werden, wobei der Sensor die Sensorwerte einer Stellung und/oder einer Drehgeschwindigkeit, einer Mittelstellung, einer Endstellung, eines Beschleunigungsprofils oder eines Drehmoments des Orthesengelenks zu verschiedenen Zeitpunkten misst, und die Sensorwerte im Speicherelement gespeichert werden.

Vorteilhafterweise kann die Bewegungsvorlage mit den im Speicherelement gespeicherten Sensorwerten abgeglichen werden. Aufgrund dieses Abgleichs der Bewegungsvorlage mit den Sensorwerten kann die Bewegungsvorlage an den Therapiefortschritt des Patienten angepasst werden und die Therapie individuell und gezielt durchgeführt werden.

In einem Beübungs-Modus zur elektronisch gesteuerten Bewegung des ersten und zweiten Halteteils relativ zueinander kann der Gelenkantrieb an das Orthesengelenk angekoppelt werden und die Steuereinheit den Gelenkantrieb gemäß der synchronisierten Bewegungsvorlage steuern.

Weiterhin kann das erfindungsgemäße Verfahren dadurch gekennzeichnet sein, dass in einem Teach-In-Modus zur Aufnahme von Sensorwerten mittels des Sensors der Gelenkantrieb von dem Orthesengelenk entkoppelt wird.

Weiterhin kann das erfindungsgemäße Verfahren dadurch gekennzeichnet sein, dass im Teach-In Modus die Halteteile zueinander bewegt werden, wobei der Sensor die Sensorwerte einer Stellung und/oder einer Drehgeschwindigkeit, einer Mittelstellung, einer Endstellung, eines Beschleunigungsprofils und/oder eines Drehmoments des Orthesengelenks zu verschiedenen Zeitpunkten misst, und die Sensorwerte im Speicherelement gespeichert werden.

Weiterhin kann das erfindungsgemäße Verfahren dadurch gekennzeichnet sein, dass im Teach-In Modus die Bewegungsvorlage mit den im Speicherelement gespeicherten Sensorwerten synchronisiert wird.

Weiterhin kann das erfindungsgemäße Verfahren dadurch gekennzeichnet sein, dass in einem Beübungs-Modus zur elektronisch gesteuerten Bewegung des ersten und zweiten Halteteils zueinander der Gelenkantrieb an das Orthesengelenk angekoppelt wird und die Steuereinheit den Gelenkantrieb gemäß der synchronisierten Bewegungsvorlage steuert.

Die oben beschriebene Orthese und die oben beschriebene Anordnung können zur therapeutischen Beübung eines Gelenks oder zur Korrektur von Fehlstellungen eines ersten Körperteils und eines mit dem ersten Körperteil durch ein Gelenk verbundenen zweiten Körperteils und/oder eines die Körperteile verbindenden Gelenks verwendet werden.

Im Folgenden werden einige Beispiele einer erfindungsgemäßen Orthese, einer erfindungsgemäßen Anordnung, eines erfindungsgemäßen Verfahrens und einer erfindungsgemäßen Verwendung gegeben. Die gezeigten einzelnen Merkmale eines Beispiels können dabei auch unabhängig vom konkreten Beispiel und in beliebiger Kombination die vorliegende Erfindung weiterbilden.

In der nachfolgenden Beschreibung der Figuren bezeichnen gleiche oder ähnliche Bezugszeichen gleiche oder ähnliche Elemente. Folgende Bezugszeichen wurden verwendet:
- 1: erfindungsgemäße Orthese
- 2: erstes Halteteil
- 3: zweites Halteteil
- 4a-c: Orthesengelenk
- 5a-c: Antriebseinheit
- 6: Potentiometer
- 7: drittes Halteteil
- 8: viertes Halteteil
- 9a-c: Schneckenantrieb
- 10a-c: Pneumatikzylinder
- 11a, b: Aktuator
- 11c, d: Pneumatischer Muskel
- 12: Befestigungssystem
- 12a: Bajonett-Verschluss
- 12b: Kugelkopfsystem
- 12c: Magnetstecksystem
- 13a: Haken
- 13b: pfannenförmiges Klemmelement
- 13c: magnetischer, zylindrischer Stift
- 14a: Aufnahme
- 14b: kugelförmiger Kopf
- 14c: magnetische Aufnahme
- 15a-c: Kolbenstange
- 16a-c: Haltestange
- 17: erstes Teil
- 18: zweites Teil
- 19a, b: Antriebsstange
- 20b1: Drahtseil
- 20b2: Umlenkrolle
- 21: Rückteil
- 22: Riemen
- 23: Riemen
- 31: Fersenkappe
- 32: Ringfassung
- 33: Ausbuchtung
- 34: Ausbuchtung
- 35: Widerstände
- 36: Verschlusselement
- 37: Unterer Lagerblock
- 41: Schiene
- 42: Schiene
- 43: Schiene
- 44: Schiene
- 45: Hinterschneidung
- 46: Kopf der Kupplungsscheibe
- 51: zylinderförmige Aussparung
- 52: Antriebsabdeckung
- 53: Grundplatte
- 54: Abtriebswelle
- 55: Kupplungshebel für Teach-In-Modus
- 56: Schrauben
- 57a, b: Durchgangsöffnungen
- 57c: Kleine Durchgangsöffnungen
- 58: Gewinde
- 59: Zahnriementrieb
- 59a, b: Synchronscheibe
- 60: Schneckenrad
- 61a, b: Fest-Los-Lagerblock
- 62: Motor
- 62a: Motorflansch
- 63: Schneckenwelle
- 64a: Trägerscheibe
- 64b: Zwölfkant-Kupplungsscheibe
- 65: Halterung des oberen Lagerblocks
- 66a, b: Durchgangsöffnungen
- 67: Drehpunkt
- 68a, b: Schrauben
- 69: Federn
- 70: Schrauben der Kupplungsscheibe
- 71: Aufnahme für Potentiometer
- 72: Druckstössel
- 73: Anschluss-Platine
- 74: Kupplungshebel-Drehachse
- 75: Oberer Lagerblock
- 76a, b: Schrauben
- 76c: Schrauben
- 77: zwölfeckige Aussparung
- 78: Aussparung
- 79: Aussparung
- 80: Bedienelement/Laptop
- 81: Energiequelle/Netzgerät
- 82: Steuerelement
- 91: Schneckenrad
- 92: Schneckenwelle
- 93: Motor
- 100: Orthese aus dem Stand der Technik

Es zeigen
- Figur 1: eine Orthese aus dem Stand der Technik;
- Figur 2: eine erfindungsgemäße Orthese mit Antriebseinheiten;
- Figur 3: eine Antriebseinheit von außen in einer Perspektivansicht;
- Figur 4: die Drehmomentübertragung auf die Schienen in verschiedenen Ansichten: a) eine Seitenansicht, b) eine Rückansicht, c) eine perspektivische Rückansicht und d) eine perspektivische Rückansicht mit nur einer Schiene;
- Figur 5: verschiedene Ansichten der Antriebseinheit ohne Gehäuse: a) eine perspektivische Vorderansicht, b) eine Vorderansicht, c) eine Rückansicht, d) eine perspektivische Rückansicht, e) eine perspektivische Unteransicht, f) eine Seitenansicht, g) eine Draufsicht und h) eine weitere Seitenansicht;

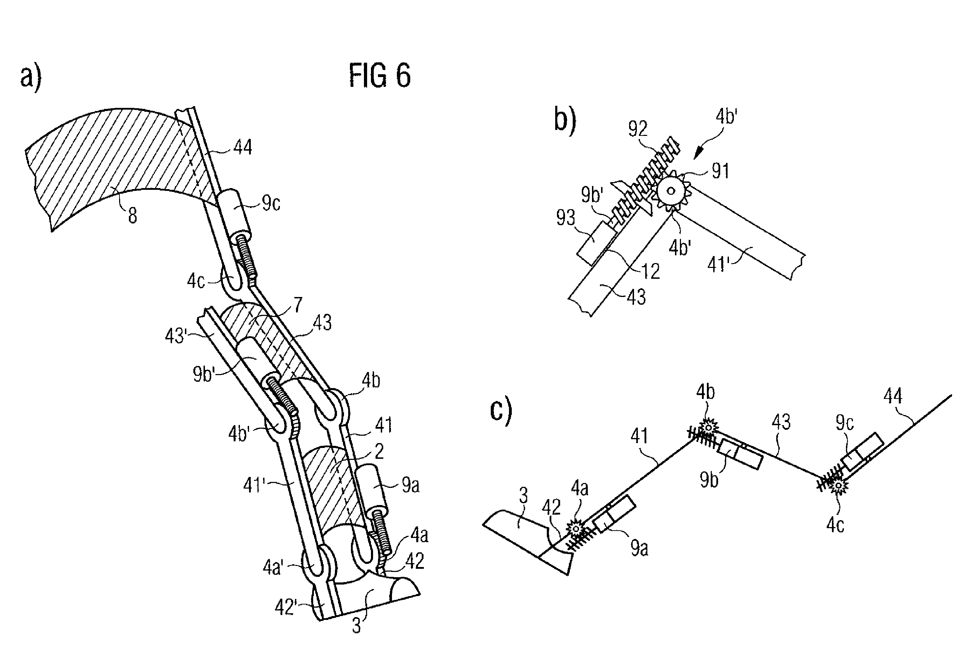

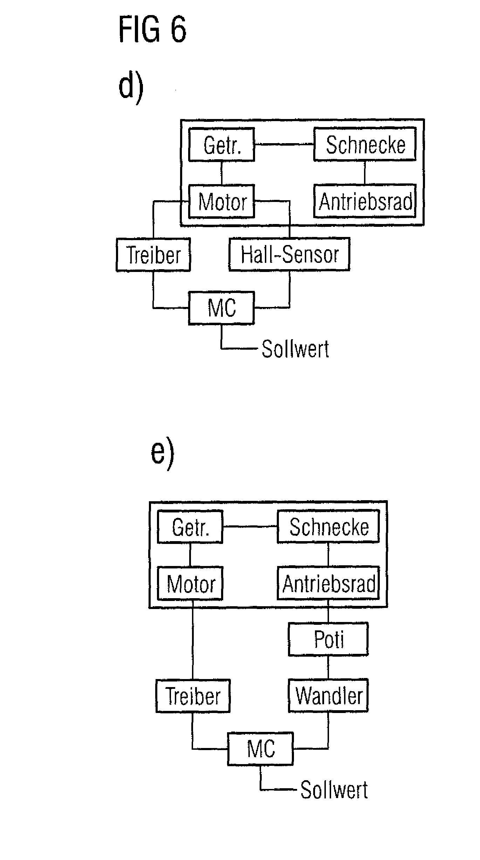

- Figur 6: eine erfindungsgemäße Orthese mit einem am Orthesengelenk befestigten Schneckenantrieb in verschiedenen Ansichten: a) eine perspektivische Vorderansicht mit drei Halteteilen und einem Schneckenantrieb, b) eine schematische Detailansicht eines Schneckenantriebs, c) eine schematische Seitenansicht mit drei Halteteilen und dem Schneckenantrieb ohne Gehäuse, d) eine schematische Darstellung einer Anordnung nach einer ersten Variante zur Beübung mit einer Orthese mit Schneckenantrieb und e) eine schematische Darstellung einer Anordnung nach einer zweiten Variante zur Beübung mit einer Orthese mit Schneckenantrieb;
- Figur 7: eine erfindungsgemäße Orthese mit drei Halteteilen und Pneumatikzylinderantrieb in verschiedenen Ansichten: a) eine perspektivische Vorderansicht, b) eine schematische Seitenansicht, c) eine schematische Draufsicht;
- Figur 8: eine erfindungsgemäße Orthese mit pneumatischem Muskel und translatorischen Aktuatoren in verschiedenen Ansichten, a) eine perspektivische Vorderansicht, b) eine schematische Seitenansicht, c) ein Schema einer Anordnung zum Betrieb der Orthese mit Pneumatikzylinderantrieb bzw. mit pneumatischem Muskel;
- Figur 9: verschiedene Mechanismen zur werkzeugfreien Befestigung der Antriebseinheit: a) einen Bajonettverschluss, b) ein Kugelkopfsystem und c) ein Magnet-Stecksystem;
- Figur 10: die Kupplungseinheit der Antriebseinheit einer erfindungsgemäßen Orthese für den Teach-In-Modus in ausgekuppeltem Zustand in verschiedenen Ansichten: a) eine perspektivische Seitenansicht, b) eine Seitenansicht, c) eine seitliche Schnittansicht, d) eine Vorderansicht, e) eine Draufsicht, f) eine schräge Schnittansicht, g) eine seitliche Schnittansicht des Schneckenrads, der Kupplungs- und der Trägerscheibe und h) eine Vorderansicht des Schneckenrads, der Kupplungs- und der Trägerscheibe;
- Figur 11: die Kupplungseinheit der Antriebseinheit einer erfindungsgemäßen Orthese für den Teach-In-Modus in eingekuppeltem Zustand in verschiedenen Ansichten: a) eine perspektivische Seitenansicht, b) eine Seitenansicht, c) eine seitliche Schnittansicht, d) eine Vorderansicht, e) eine Draufsicht, f) eine schräge Schnittansicht, g) eine seitliche Schnittansicht des Schneckenrads, der Kupplungs- und der Trägerscheibe, h) eine Vorderansicht der Elemente aus g), und i) eine Schnittansicht der Elemente aus g) entlang der Fläche zwischen Schneckenrad und Trägerscheibe;
- Figur 12: eine Anordnung zum Betrieb einer Orthese mit einer Antriebseinheit.

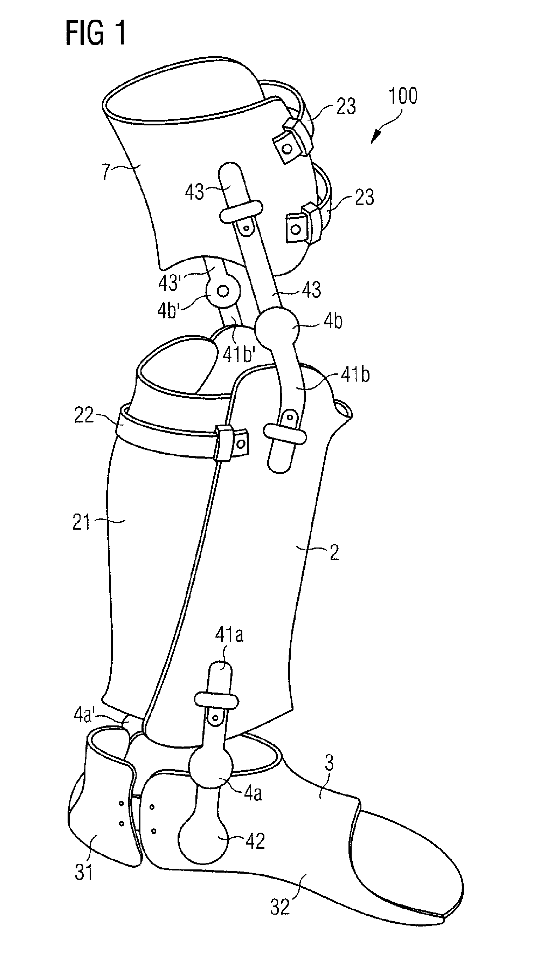

Figur 1 zeigt eine dreiteilige bilaterale Beinorthese 100 wie sie aus dem Stand der Technik bekannt ist. Die Orthese 100 weist ein erstes Halteteil 2 für einen Unterschenkel, ein zweites Halteteil 3 für den Fuß sowie ein drittes Halteteil 7 für einen Oberschenkel auf. Das erste Halteteil 2 ist mit dem zweiten Halteteil 3 über ein rechtes Orthesengelenk 4a und ein linkes Orthesengelenk 4a' verbunden. Das Orthesengelenk 4a verbindet Schienen 42 und 41a an jeweils einem Ende der Schienen miteinander, sodass die Schienen 42 und 41a zueinander drehbar sind. Die Schiene 42 ist dabei mit ihrem dem Orthesengelenk 4a abgewandten Ende an der rechten Außenseite des zweiten Halteteils 3 befestigt. Mit Außenseite der Orthese oder Außenseite eines Halteteils ist hier und im Folgenden die Seite der Orthese bzw. des Halteteils gemeint, welche sich auf der dem von der Orthese aufgenommen Körperteil abgewandten Seite der Orthese bzw. des Halteteils befindet. Die Schiene 41a ist mit ihrem dem Orthesengelenk 4a abgewandten Ende an der rechten Außenseite des ersten Halteteils 2 befestigt. Das Orthesengelenk 4a' verbindet Schienen 42' und 41a' (in Figur 1 nicht dargestellt) an jeweils einem Ende der Schienen miteinander, sodass die Schienen 42' und 41a' zueinander drehbar sind. Die Schiene 42' ist dabei mit ihrem dem Orthesengelenk 4a' abgewandten Ende an der linken Außenseite des zweiten Halteteils 3 befestigt, während die Schiene 41a' mit ihrem dem Orthesengelenk 4a' abgewandten Ende an der linken Außenseite des ersten Halteteils 2 befestigt ist.

Das erste Halteteil 2 und das dritte Halteteil 7 sind über ein rechtes Orthesengelenk 4b und ein linkes Orthesengelenk 4b' verbunden. Das Orthesengelenk 4b verbindet Schienen 41b und 43 an jeweils einem Ende der Schienen miteinander, sodass die Schienen 41b und 43 zueinander drehbar sind. Die Schiene 41b ist mit ihrem dem Orthesengelenk 4b abgewandten Ende an der rechten Außenseite des ersten Halteteils 2 befestigt. Die Schiene 43 ist mit ihrem dem Orthesengelenk abgewandten Ende an der rechten Außenseite des dritten Halteteils 7 befestigt. Das Orthesengelenk 4b' verbindet Schienen 41b' und 43' an jeweils einem Ende miteinander, sodass die Schienen 41b' und 43' zueinander drehbar sind. Dabei ist die Schiene 41b' mit ihrem dem Orthesengelenk 4b' abgewandten Ende an der linken Außenseite des ersten Halteteils 2 befestigt, und die Schiene 43' mit ihrem dem Orthesengelenk 4b' abgewandten Ende an der rechten Außenseite des dritten Halteteils 7 befestigt.

Die Schienen 41a, 41a', 41b, 41b', 43 und 43' sind mittels eines Verschlusssystems von der Orthese 100 abnehmbar, während die Schienen 42 und 42' fest mit der Orthese 100 verbunden sind.

Im Mittelfußbereich weist das zweite Halteteil 3 eine Ringfassung 32 auf, die im Zehenbereich offen ist. Im Fersenbereich weist das zweite Halteteil 3 eine Fersenkappe 31 auf, die sowohl verstellbar als auch öffenbar und verschließbar ist. Das erste Halteteil 2 weist ferner im Wadenbereich ein Rückteil 21 auf, welches mittels eines Riemens 22 verstellbar, öffenbar und verschließbar ist. Das dritte Halteteil 7 ist mittels Riemen 23 in der Weite verstellbar, öffenbar und verschließbar. Die Riemen 23 sind auf der Vorderseite des dritten Halteteils 7 angeordnet, wobei der Vorderseite der Orthese bzw. eines Halteteils die Seite der Orthese bzw. des Halteteils entspricht, die im gestreckten Zustand dem Zehenbereich des zweiten Halteteils 3 zugewandt ist.

Wird die Orthese 100 an ein Bein angelegt, dann umschließt das dritte Halteteil 7 den mittleren Teil des Oberschenkels, wobei durch die Riemen 23 die Weite des Halteteils 7 angepasst werden kann. Das erste Halteteil 2 umschließt den Unterschenkel vom Knie bis zum Sprunggelenk. Im Wadenbereich kann die Weite des ersten Halteteils 2 durch Einstellen der Position des Rückteils 21 mittels des Riemens 22 angepasst werden. Im zweiten Halteteil 3 wird der Fuß von der Ringfassung 32 im Mittelfußbereich umschlossen. Im Zehenbereich ist das zweite Halteteil 3 offen. Im Fersenbereich lässt sich mittels der verstellbaren Fersenkappe 32 die Länge des ersten Halteteils an den Fuß anpassen. Die Orthesengelenke 4b und 4b' befinden sich nach Anlegen der Orthese rechts und links im Bereich des Knies, sodass sich die Halteteile 7 und 2 mit der Bewegung des Kniegelenks zueinander bewegen lassen. Die Orthesengelenke 4a und 4a' befinden sich dann rechts und links im Bereich des Sprunggelenks, sodass sich die Halteteile 2 und 3 mit der Bewegung des Sprunggelenks zueinander bewegen lassen.

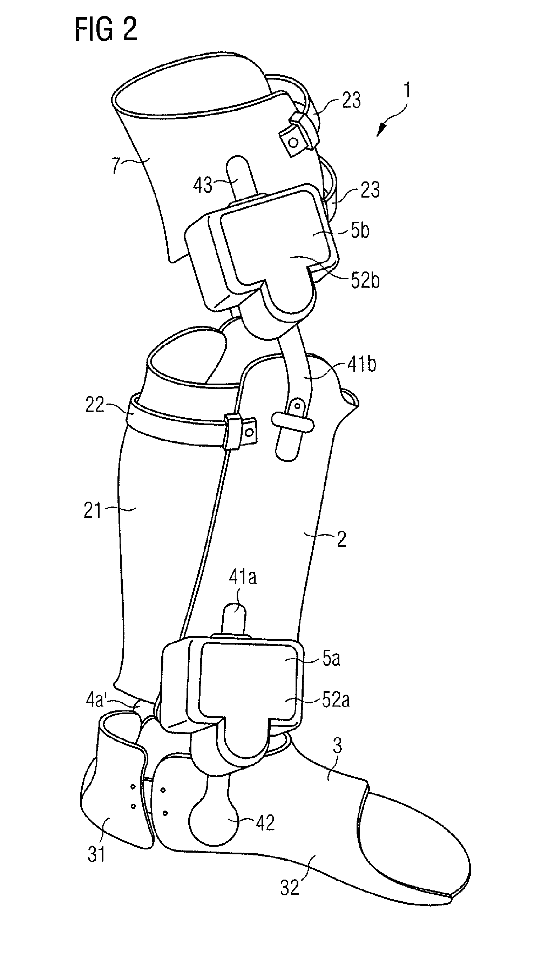

Figur 2 zeigt eine Beinorthese 1 gemäß der vorliegenden Erfindung. Die Orthese 1 weist alle Merkmale der Orthese 100 aus Figur 1 auf. Zusätzlich sind an den Orthesengelenken 4a und 4b Antriebseinheiten 5a und 5b monolateral, also nur auf einer Seite der Orthese, angeordnet. Auch eine Orthese mit bilateral, also beidseitig der Orthese, angeordneten Antriebseinheiten ist eine mögliche Ausgestaltung der Erfindung. Die Antriebseinheiten 5a und 5b weisen Antriebsabdeckungen 52a und 52b auf, die hier beispielsweise kastenförmig ausgebildet sind. Es ist jedoch auch möglich die Antriebsabdeckungen 52a und 52b in einer anderen Form zu gestalten.

In den weiteren Figuren werden der Aufbau und die Funktionsweise der Antriebseinheiten 5a und 5b der Orthese 1 meist an dem konkreten Beispiel der Antriebseinheit 5a erläutert. Die Antriebseinheit 5b ist jedoch völlig analog aufgebaut.

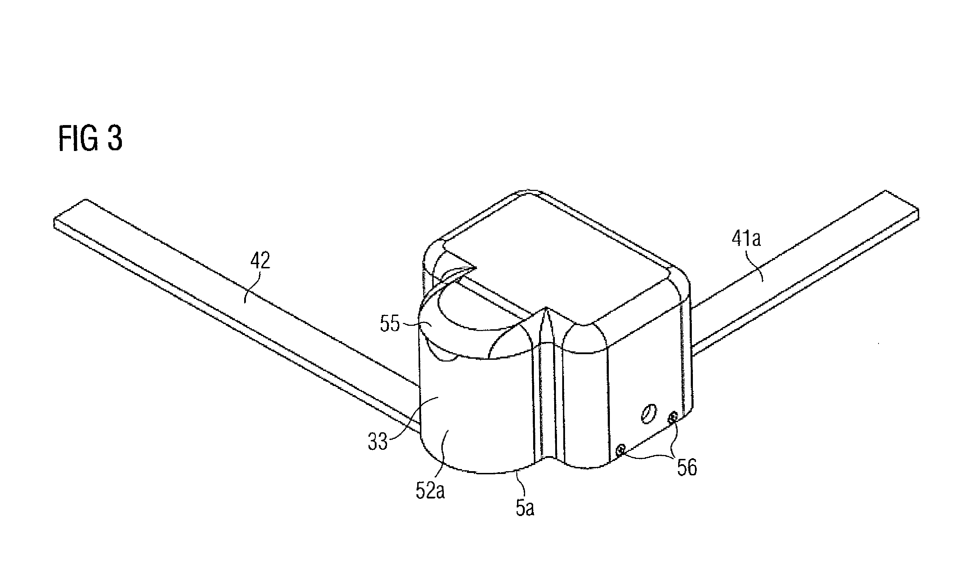

Figur 3 zeigt die Antriebseinheit 5a mit der Schiene 41a und der Schiene 42 in einer Perspektivansicht. Im Vordergrund ist die Antriebsabdeckung 52a der Antriebseinheit 5a zu sehen. Die Antriebsabdeckung 52a hat hier in etwa eine Quaderform, wobei auf einer der langen Seiten des Quaders eine Ausbuchtung 33 in Form eines halben, auf seiner Grundfläche stehenden Zylinders hervorsteht. Auf der Seite der Ausbuchtung 33 ragt die Schiene 42 unter der Antriebseinheit 5a hervor. Auf der der Ausbuchtung 33 gegenüberliegenden Seite der Antriebseinheit 5a ragt die Schiene 41a hervor. Die Schienen 41a und 42 sind Teil des Orthesengelenks 4a, welches in Figur 3 von der Antriebsabdeckung 52a verdeckt wird und in Figur 1 an der Orthese 100 im Bereich des Sprunggelenks angeordnet ist.

In dem der Schiene 42 abgewandten Bereich der Ausbuchtung 33 weist die Antriebseinheit 5a einen Kupplungshebel 55 auf, welcher formschlüssig mit der Abdeckung 52a abschließt und zum Auskuppeln des Gelenkantriebs während des Teach-In-Modus dient. An den kurzen Seiten und in dem der Schiene 41a zugewandten Bereich der quaderförmigen Antriebsabdeckung 52a sind Befestigungsschrauben 56 angeordnet, mit denen die Antriebsabdeckung 52a an einer Grundplatte 53 (in Figur 3 nicht dargestellt) befestigt wird.

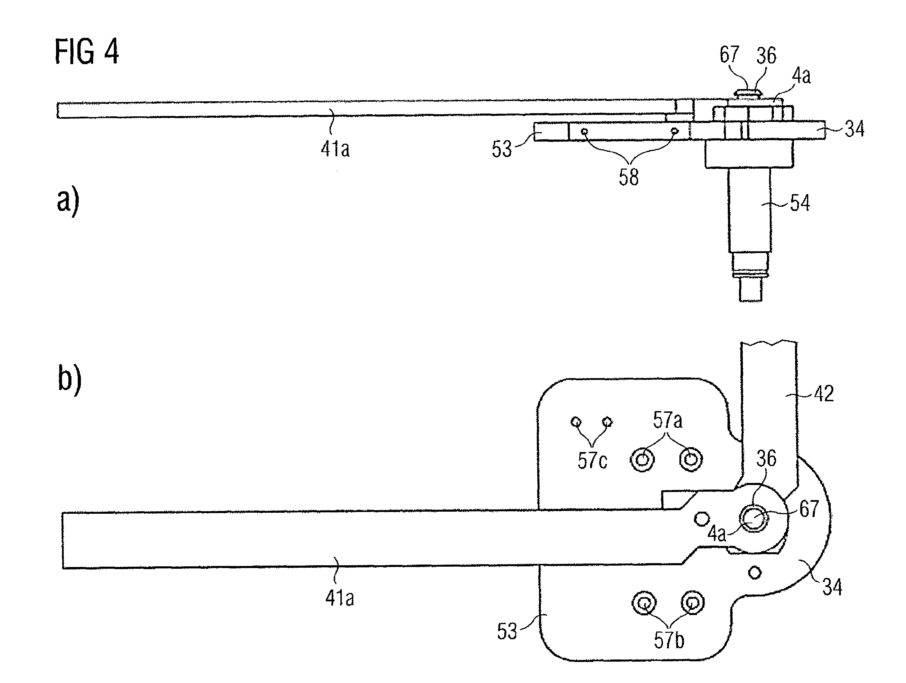

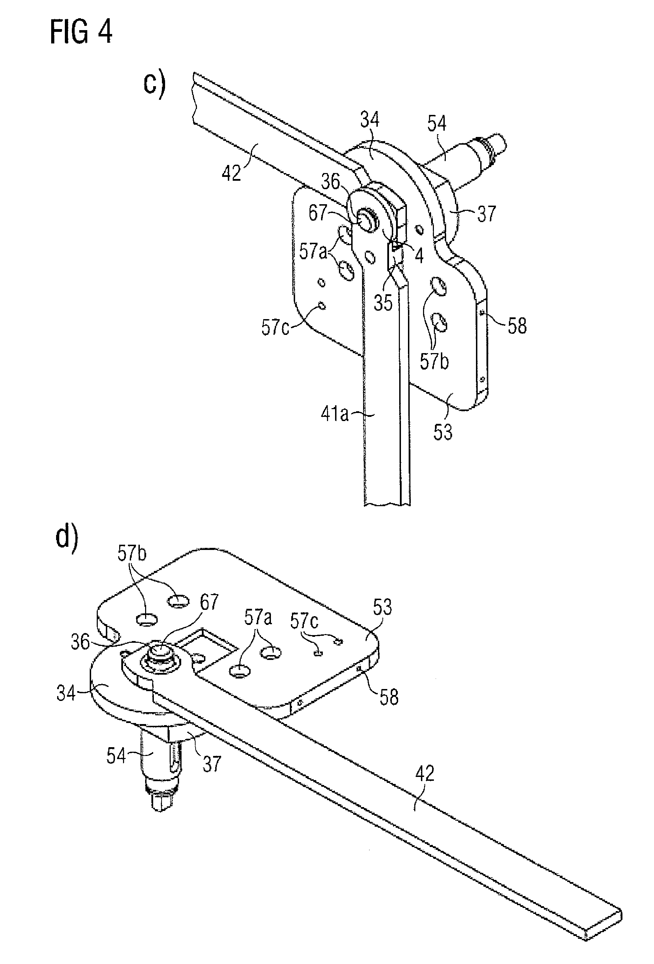

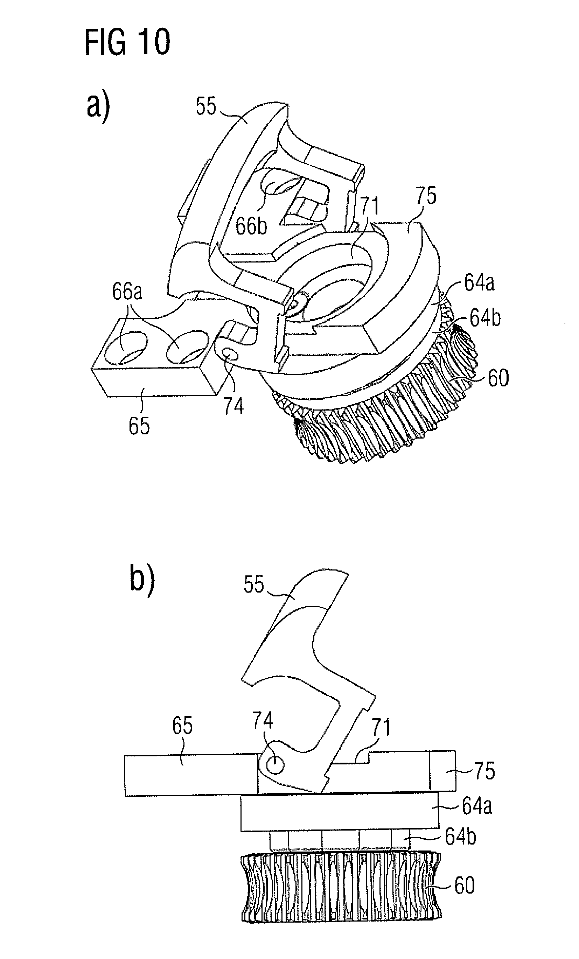

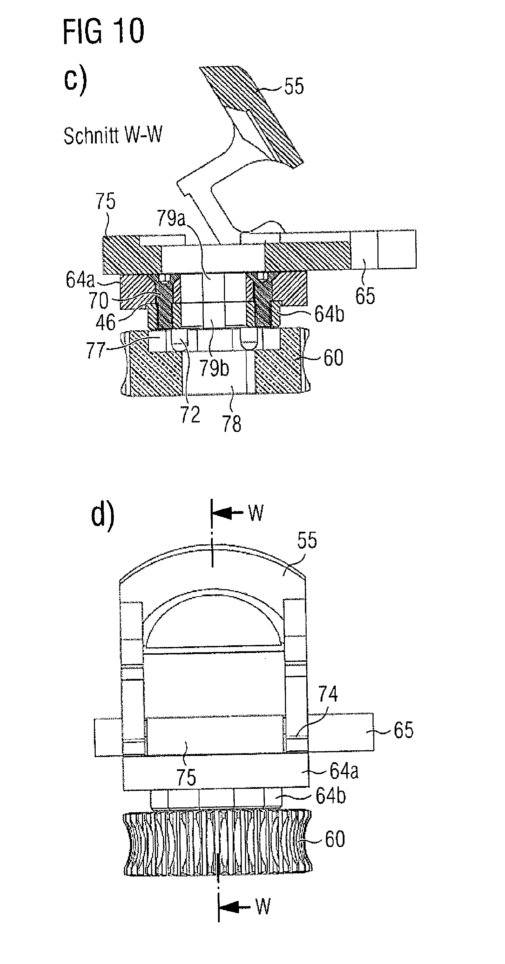

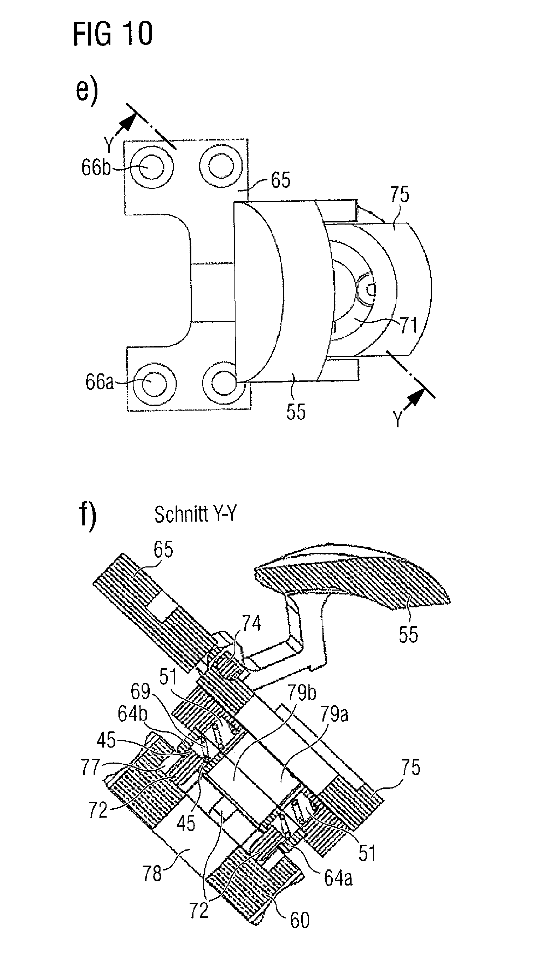

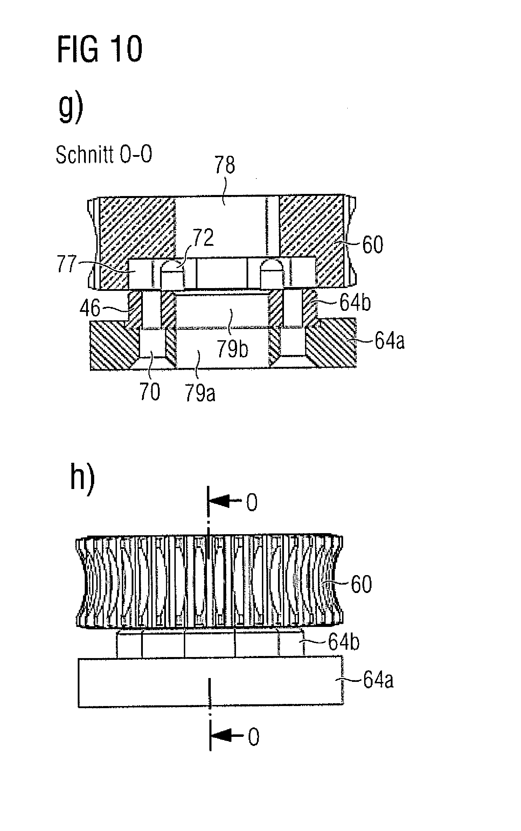

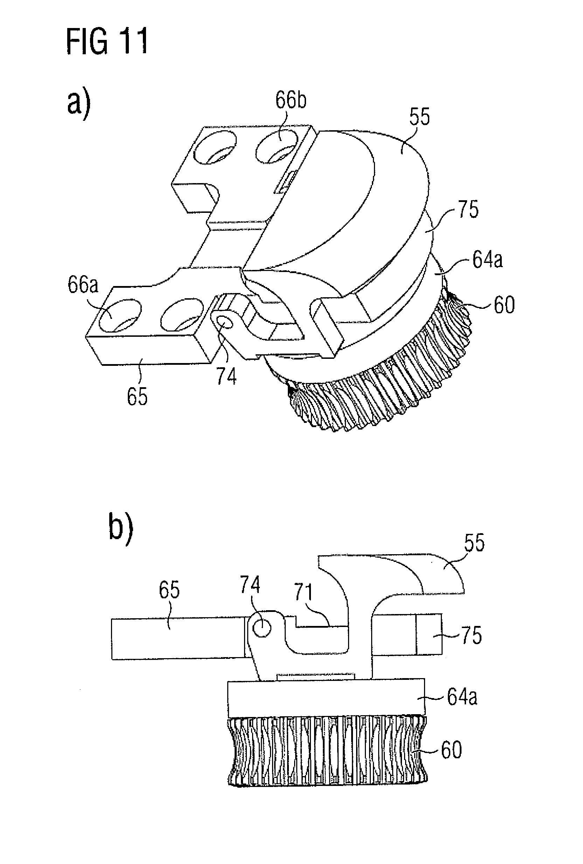

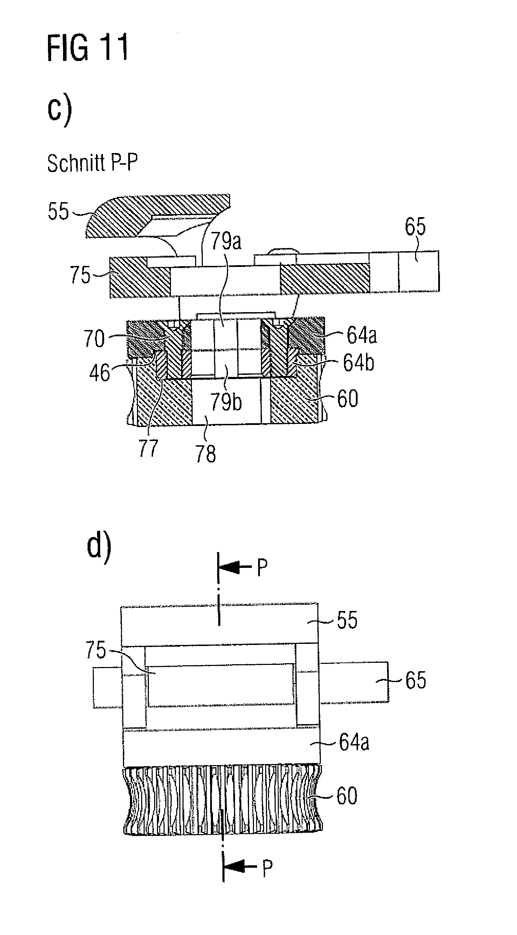

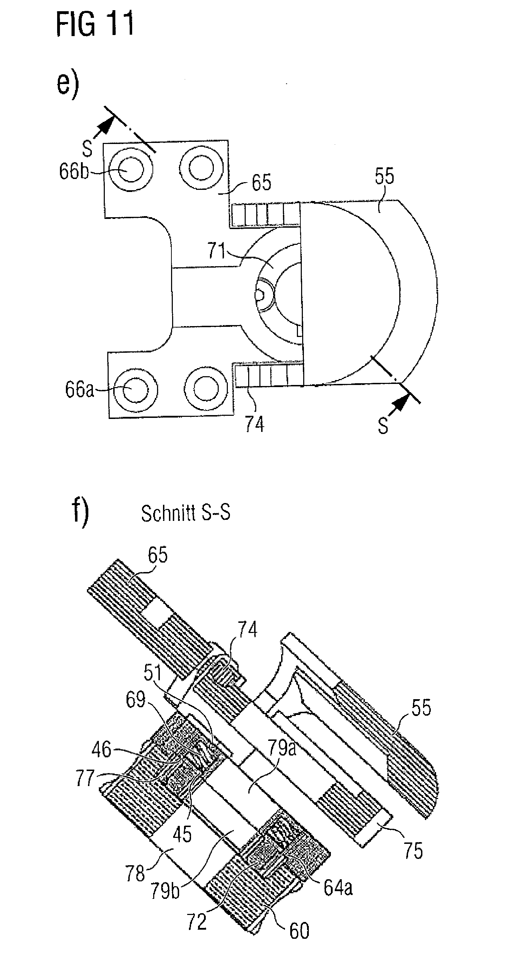

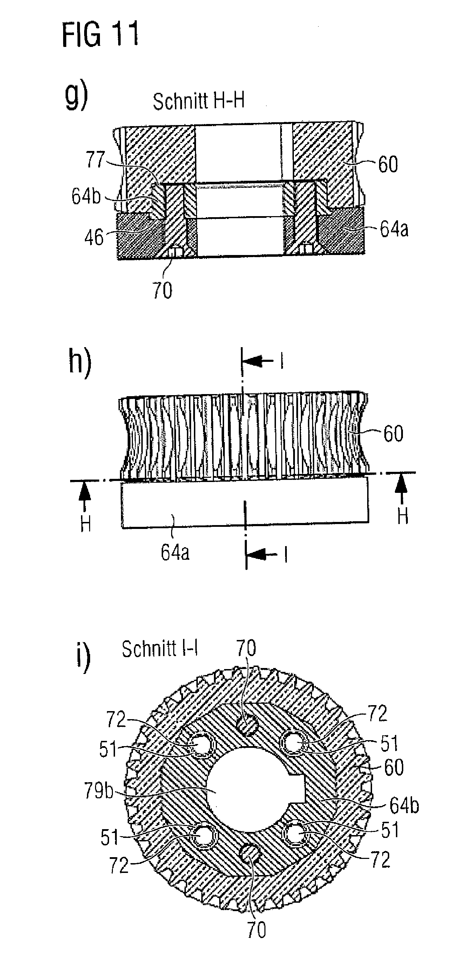

Figur 4 zeigt eine Abtriebswelle 54, das Orthesengelenk 4a sowie die Schiene 41a und Schiene 42 in verschiedenen Ansichten. In Figur 4 a) sind die Abtriebswelle 54, das Orthesengelenk 4a und die Schiene 41a in einer Seitenansicht zu sehen, während Figur 4 b) eine Unteransicht zeigt. Sowohl die Schienen 41a und 42 als auch die Abtriebswelle 54 sind Bestandteile des Orthesengelenks 4a. Die Enden der Schienen 41a und 42, an denen die Schienen 41a und 42 miteinander drehbar verbunden sind, sind ringförmig ausgebildet mit einer Öffnung, durch welche das Ende der Abtriebswelle 54 läuft, welches im an die Orthese 1 montierten Zustand wie in Figur 2 der Orthese 1 zugewandt ist. Die Abtriebswelle 54 liegt parallel zur Drehachse des Orthesengelenks 4a, welche durch den Drehpunkt 67 und senkrecht zu den Schienen 41a und 42 verläuft. Die Abtriebswelle 54 ist formschlüssig mit der ringförmigen Öffnung der Schiene 42 verbunden, kann sich jedoch in der ringförmigen Öffnung der Schiene 41a drehen. Auf der der gedachten Orthese abgewandten Seite der Schienen 41a und 42 befindet sich eine Grundplatte 53 der Antriebseinheit 5a, durch welche die Abtriebswelle 54 ebenfalls läuft. Die Grundplatte 53 ist im Wesentlichen rechteckig ausgebildet mit einer halbkreisförmigen Ausbuchtung 34 im Bereich der Abtriebswelle 54. Ein an der Abtriebswelle 54 angeordnetes Verschlusselement 36 und ein unterer Lagerblock 37 sichern die Schienen 42, 41a und die Grundplatte 53 gegen translatorische Bewegung in einer Richtung parallel zur Drehachse der Abtriebswelle 54. Der Lagerblock 37 enthält ein Rillenkugellager, durch welches die Abtriebswelle 54 in dem unteren Lagerblock 37 drehbar gelagert ist. Bei dem Verschlusselement 36 kann es sich beispielsweise um einen Sicherungsring oder um ein anderes Element handeln. Die Schiene 41a ist parallel zu den kurzen Seiten der rechteckigen Grundplatte 53 ausgerichtet und durch Widerstände 35 formschlüssig mit der Grundplatte 53 verbunden. Die Widerstände sind senkrecht an der Grundplatte 53 und parallel an gegenüberliegenden Seiten der Schiene 41a anliegend im Bereich der Abtriebswelle 54 angeordnet. Dadurch wird ein Verdrehen der Schiene 41a in einer ebene parallel zur Grundplatte 53 und gegen die Grundplatte 53 verhindert. Auf der der Orthese abgewandten Seite der Grundplatte 53 befindet sich der Teil der Abtriebswelle 54, welcher ausgebildet ist, um die Kupplungseinheit, welche in den Figuren 10 und 11 dargestellt ist, aufzunehmen.

An den kurzen Seiten der rechteckigen Grundplatte 53, die parallel zur Schiene 41a verlaufen, befinden sich Gewinde 58, die zur Befestigung der Antriebsabdeckung 52a mit den Befestigungsschrauben 56 vorgesehen sind.

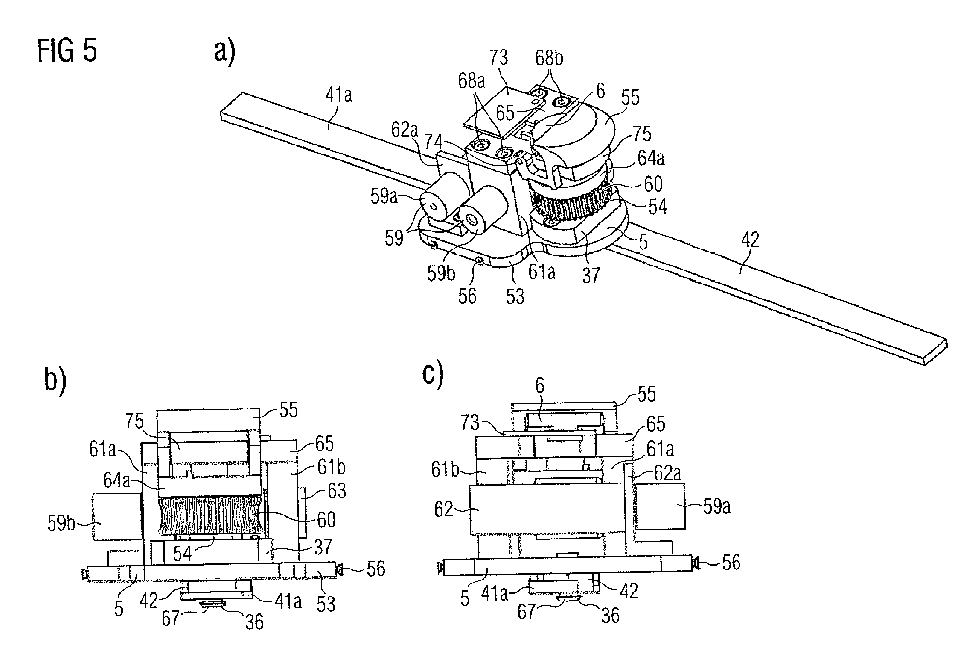

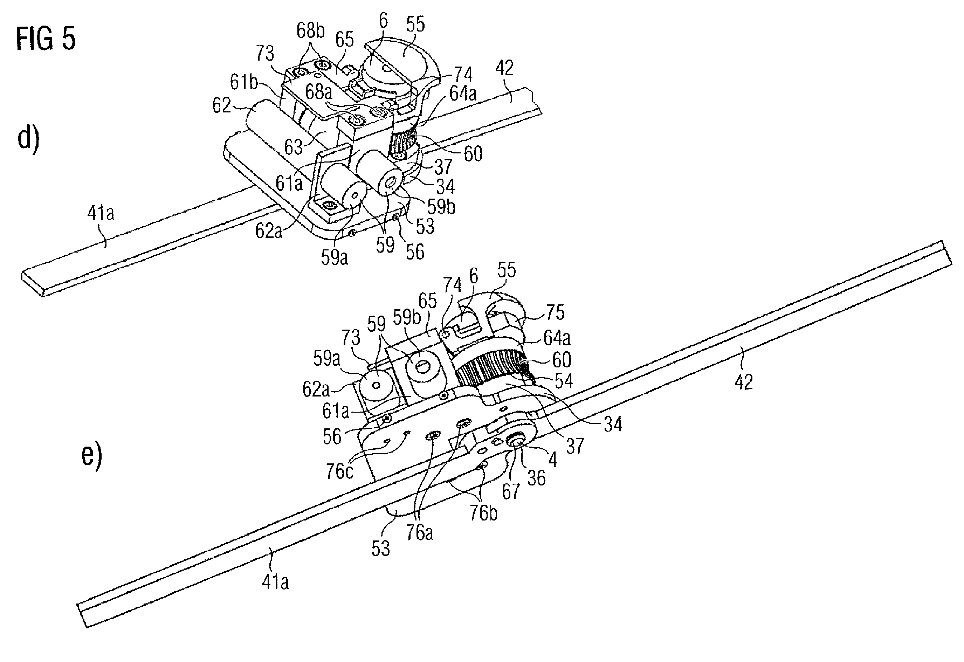

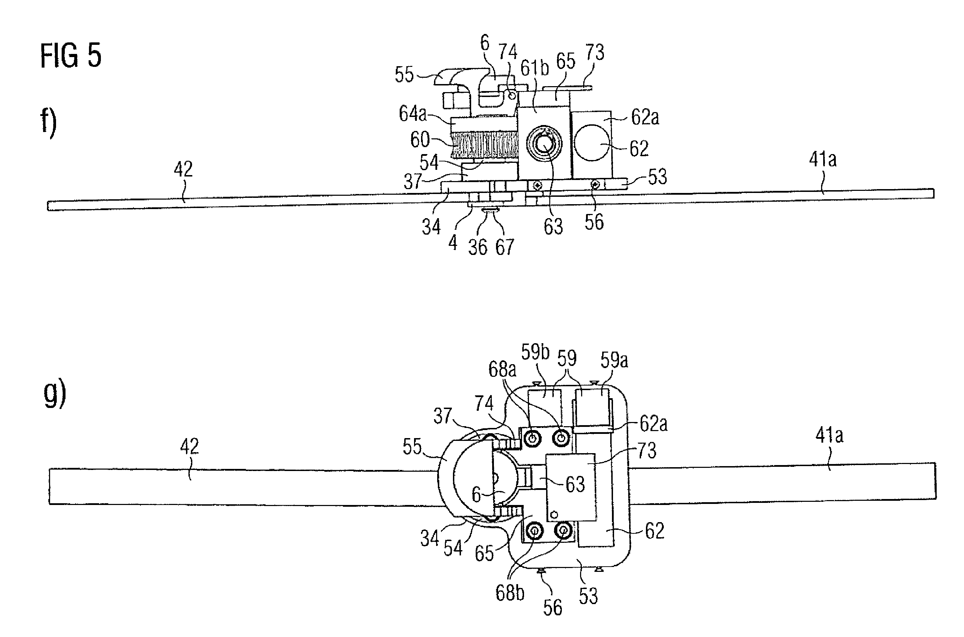

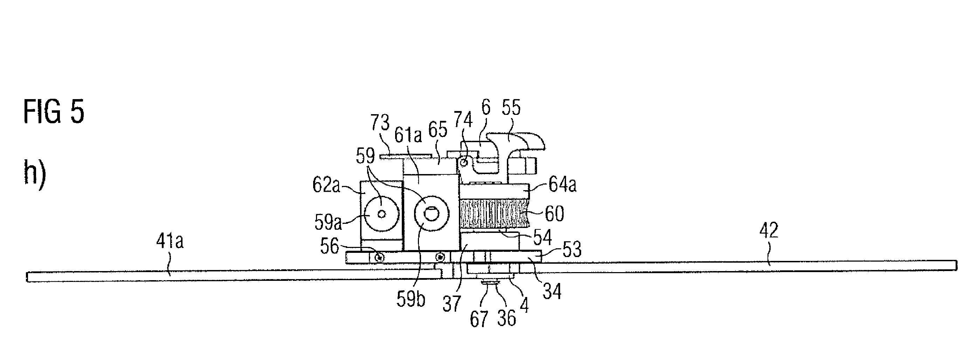

In Figur 4 b) sind darüber hinaus auf der Unterseite der Grundplatte 53 auf beiden Seiten der Schiene 41a je zwei Durchgangsöffnungen 57a und 57b zur Befestigung von Fest-Los-Lagerblöcken 61a und 61b (vgl. Figur 5 a) bis h)) auf der Grundplatte 53 angeordnet, welche zu der der Unterseite der Grundplatte 53 abgewandten Oberseite der Grundplatte 53 hin eine Verjüngung aufweisen. Zusätzlich sind zwei kleine Durchgangsöffnungen 57c, welche einen geringeren Durchmesser aufweisen als die Durchgangsöffnungen 57a und 57b, benachbart zu den Durchgangsöffnungen 57a an der Unterseite der Grundplatte 53 zur Befestigung eines Motorflansches 62a (vgl. Figur 5 a) bis h)) angeordnet. Die Durchgangsöffnungen 57c weisen wie die Durchgangsöffnungen 57a und 57b eine Verjüngung zur Oberseite der Grundplatte 53 hin auf.

Figur 4 c) zeigt die Grundplatte sowie die Schiene 41a und die Schiene 42 in einer weiteren Unteransicht.

Figur 4 d) zeigt die Grundplatte 53 mit der Schiene 42, jedoch ohne die Schiene 41a, in einer weiteren Unteransicht.

Figuren 5 a) bis h) zeigen die Antriebseinheit 5a verbunden mit der Schiene 41a und der Schiene 42 in verschiedenen Ansichten. Direkt auf den Schienen 41a und 42 befindet sich die Grundplatte 53, die weitestgehend rechteckig ausgebildet ist mit abgerundeten Ecken und an der Seite, welche die Schiene 42 kreuzt, eine halbkreisförmige Ausbuchtung 34 aufweist. An den Seiten der Grundplatte 53, die keine der Schienen 41a oder 42 kreuzen, sind Schrauben 56 zur Befestigung der Antriebsabdeckung 52a vorhanden. Der weitgehend rechteckige Bereich der Grundplatte 53, im Weiteren auch hinterer Bereich genannt, beherbergt einen Motorflansch 62a und eine Fest-Los-Lagerung, welche zwei Lagerblöcken 61a und 61b aufweist. Der Motorflansch 62a ist auf die Oberseite der Grundplatte 53 aufgeschraubt und dient der Halterung des Motors 62. Die Fest-Los-Lagerblöcke 61a und 61b sind ebenfalls auf die Oberseite der Grundfläche 53 aufgeschraubt und weisen jeweils ein Rillenkugellager auf. In diesen Lagerblöcken 61a und 61b mit Rillenkugellager ist eine Schneckenwelle 63 drehbar gelagert, wobei einer der Lagerblöcke zur Fest-Lagerung der Schneckenwelle zusätzlich einen Sprengring aufweist, welcher die Schneckenwelle an einer Bewegung in Richtung der Drehachse der Schneckenwelle hindert. Der Motor 62 ist zylinderförmig ausgebildet, und der Motor 62 und die Schneckenwelle 63 liegen parallel zueinander sowie zu der Seite der Grundplatte 53, welche die Schiene 41a kreuzt, also zu der der Ausbuchtung 34 abgewandten Seite der Grundplatte 53. Zu einer Seite der Grundplatte 53 hin, an welcher sich Schrauben 56 befinden, ist ein Zahnriementrieb 59 angeordnet, durch welchen der Motor 62 eine Drehbewegung auf die Schneckenwelle 63 überträgt. Zum Zahnriementrieb 59 gehören zwei zueinander drehbare zylinderförmige Synchronscheiben 59a und 59b, wobei die Synchronscheibe 59a mit dem Motor 62 derart fest verbunden ist, dass die Symmetrieachse der Synchronscheibe 59a mit der Drehachse des Motors 62 identisch ist und der Motor 62 eine Drehbewegung auf die Synchronscheibe 59a überträgt, so dass diese sich um ihre Symmetrieachse dreht. Die Synchronscheibe 59b ist mit der Schneckenwelle 63 derart fest verbunden, dass die Drehachse der Schneckenwelle 63 mit der Symmetrieachse der Synchronscheibe 59b identisch ist und die Schneckenwelle 63 eine Drehbewegung auf die Synchronscheibe 59b überträgt, so dass diese sich um ihre Symmetrieachse dreht. Die Synchronscheiben 59a und 59b weisen jeweils eine durch das Übersetzungsverhältnis des Zahnriementriebs definierte Anzahl an Zähnen (nicht dargestellt) auf ihrer Mantelfläche auf. Die Synchronscheiben 59a und 59b sind ferner über einen Zahnriemen (nicht dargestellt) miteinander gekoppelt, wobei die Zähne des Zahnriemens in die Vertiefungen zwischen jeweils zwei Zähnen der Synchronscheiben 59a und 59b greifen. Der Motor 62 kann somit eine Drehbewegung um seine Drehachse über die beiden Synchronscheiben 59a und 59b und den Zahnriemen des Zahnriementriebs 59 auf die Schneckenwelle 63 übertragen.

Alternativ zu einem Zahnriementrieb kann auch ein Zahnradantrieb verwendet werden. In diesem Fall weisen die Synchronscheiben einen größeren Durchmesser als beim Zahnriementrieb auf, sodass die Zähne der einen Synchronscheibe direkt zwischen die Zähne der zweiten Synchronscheibe greifen. Die Drehbewegung des Motors 62 wird beim Zahnradantrieb direkt von der Synchronscheibe 59a auf die Synchronscheibe 59b und damit auf die Schneckenwelle 63 übertragen.

Im Bereich der halbkreisförmigen Ausbuchtung der Grundplatte 53 befindet sich die Drehachse des Orthesengelenks 4a. Unterhalb dieses Bereiches der Grundplatte 53, der im Folgenden auch vorderer Bereich genannt wird, befindet sich der Drehpunkt 67 des Orthesengelenks. In diesen Drehpunkt 67 mündet senkrecht zu den Schienen 41a und 42 die Abtriebswelle 54. Die Abtriebswelle 54 läuft ebenfalls durch eine Öffnung in der Ausbuchtung 34 der Grundplatte 53. Die Grundplatte 53 ist jedoch nicht fest mit der Abtriebswelle 54 gekoppelt, sondern frei um diese in Drehrichtung des Orthesengelenks 4a drehbar. Auf der der Grundplatte 53 abgewandten Seite der Schienen 41a und 42 befindet sich das Verschlusselement 36, welches die Schienen 41a und 42 am Abrutschen von der Abtriebswelle 54 hindert. Auf die Abtriebswelle 54 sind nun auf der den Schienen 41a und 42 abgewandten Seite der Grundplatte 53 nacheinander verschiedene Bestandteile der Antriebseinheit 5a aufgeschoben. Auf der Grundplatte 53 befindet sich zunächst der untere Lagerblock 37, welches zusammen mit dem Verschlusselement 36 die Schienen 41a und 42 sowie die Grundplatte 53 an einer translatorischen Bewegung in einer Richtung parallel zur Drehachse der Abtriebswelle 54 auf der Abtriebswelle 54 hindert. Der untere Lagerblock 37 weist ein Rillenkugellager auf, durch welches die Abtriebswelle 54 in dem unteren Lagerblock 37 drehbar gelagert ist. Hierauf ist ein Schneckenrad 60 aufgeschoben, welches prinzipiell unabhängig von der Abtriebswelle 54 frei drehbar auf der Abtriebswelle 54 gelagert ist und mit der benachbarten Schneckenwelle 63 in Kontakt steht. Auf das Schneckenrad 60 folgt eine 12-Kant-Kupplungsscheibe 64b, die fest mit einer darüber liegenden Trägerscheibe 64a verbunden ist. In den Figuren 5 a) bis h) ist die Kupplungsscheibe 64b nicht sichtbar, da diese in den der Trägerscheibe 64a zugewandten Bereich des Schneckenrads 60 formschlüssig eingelassen ist. Die Trägerscheibe 64a ist in Drehrichtung des Orthesengelenks 4a fest mit der Abtriebswelle 54 gekoppelt, sodass die Trägerscheibe 64a eine Drehbewegung um die Drehachse des Orthesengelenks 4a auf die Abtriebswelle 54 übertragen kann. In einer Richtung parallel zur Drehachse ist die Trägerscheibe 64a jedoch verschiebbar. Auf der hier sichtbaren Trägerscheibe 64a befinden sich weiter ein oberer Lagerblock 75, welcher die Abtriebswelle 54 auf der der Kupplungsscheibe 64b abgewandten Seite der Trägerscheibe 64a in Achsrichtung der Abtriebswelle 54 sichert. Der obere Lagerblock 75 weist ein Rillenkugellager auf, durch welches die Abtriebswelle 54 drehbar in dem oberen Lagerblock 75 gelagert ist. Der obere Lagerblock 75, welcher sich in der Ausbuchtung 33 (vgl. Figur 3) befindet, weist eine Halterung 65 auf, mit welcher dieser einstückig verbunden ist. Die Halterung 65 erstreckt sich im quaderförmigen Bereich der Antriebseinheit 54 über der Schneckenwelle 63. Über die Halterung 65 ist der obere Lagerblock 75 auf den Fest-Los-Lagerblöcken 61a und 61b mit jeweils zwei Schrauben 68a und 68b befestigt. Dabei verschwinden die Schrauben 68a und 68b in den Durchgangsöffnungen 66a und 66b in der Halterung 65 (vgl. Figur 10a und 11a), so dass die Köpfe der Schrauben 68a und 68b bündig mit der den Fest-Los-Lagerblöcken 61a und 61b abgewandten Oberseite der Halterung 65 abschließen. Die Durchgangsöffnungen 66a und 66b weisen zu den Lagerblöcken 61a und 61b hin Verjüngungenen auf, an denen sich die Schrauben 68a und 68b abstützen.
An der Schnittfläche, an welcher sich die Halterung 65 des oberen Lagerblocks 75 an den oberen Lagerblock 75 anschließt, ist an zwei gegenüberliegenden Seiten der Schnittfläche ein Kupplungshebel 55 für den Teach-In-Modus drehbar angebracht, welcher um eine Drehachse 74, die in der Schnittfläche verläuft, drehbar ist. Über den Kupplungshebel 55 lässt sich der Gelenkantrieb von der Orthese ein- und auskuppeln. Die Funktion des Ein- und Auskuppelns wird in der Beschreibung der Figuren 10 genauer erläutert. Die in den Figuren 5 a) bis h) dargestellte Antriebseinheit 5a befindet sich im eingekuppelten Zustand, weshalb die Kupplungsscheibe 64b nicht sichtbar, sondern in das Schneckenrad 60 eingelassen ist. Dementsprechend befindet sich der Kupplungshebel 55 in geschlossener Position und in Kontakt mit der Trägerscheibe 64a. Zum Öffnen des Kupplungshebels 55 wird dieser nach oben gezogen oder gedrückt, wobei sich der Kupplungshebel 55 um die Achse 74 dreht. Zwischen der Halterung 65, dem Kupplungshebel 55 und dem oberen Lagerblock 75 über der Trägerscheibe 64a befindet sich ein Potentiometer 6, welches mit der Abtriebswelle 54 derart verbunden ist, dass über die Stellung der Abtriebswelle die Winkelstellung des Orthesengelenks 4a messbar ist. Auf der Halterung 65 des oberen Lagerblocks 75 und somit oberhalb der Fest-Los-Lagerblöcke 61a und 61b befindet sich eine Platine 73, welche elektrische Anschlüsse zum Anschließen von externen sowie in der Antriebseinheit 5a enthaltenen elektrischen Komponenten, beispielsweise für den in der Antriebseinheit 5a enthaltenen Motor 62, enthält.

Das Funktionsprinzip der Antriebseinheit 54 ist Folgendes: Der Motor 62 überträgt über den Zahnriementrieb 59 eine Drehbewegung auf die Schneckenwelle 63. Die Schneckenwelle 63 überträgt die Drehbewegung ihrerseits auf das anliegende Schneckenrad 60. Über die Kupplungsscheibe 64b, die formschlüssig in den der Trägerscheibe 64a zugewandten Bereich des Schneckenrads 60 eingelassen ist, überträgt das Schneckenrad 60 seine Drehbewegung an die Trägerscheibe 64a, welche ihrerseits die Drehbewegung auf die Abtriebswelle 54 überträgt. Mit der Drehbewegung der Abtriebswelle 54 bewegt sich nun die mit der Abtriebswelle 54 fest verbundene Schiene 42 um die Drehachse des Orthesengelenks 4a und gegen die Schiene 41a, die in Drehrichtung des Orthesengelenks 4a fest mit der Grundplatte 53 gekoppelt ist. Die Schienen 41a und 42 übertragen diese Relativbewegung zueinander auf das erste und zweite Halteteil 2 und 3.

Figur 5 a) zeigt die Antriebseinheit 5a in einer seitlichen Perspektivansicht mit Blick auf den Kupplungshebel 55, die Halterung 65 und die Platine 73. Ferner sind die Synchronscheiben 59a und 59b zu sehen, die jeweils am Motor 62 und der Schneckenwelle 63 zueinander drehbar angeordnet sind.

Figur 5 b) zeigt nun die Antriebseinheit 5a aus Figur 5 a) in einer Vorderansicht, wobei das Schneckenrad 60 und der Kupplungshebel 55 im Vordergrund zu sehen sind. Im Vordergrund ist die gesamte Kupplungseinheit zum Ein- und Ausschalten des Teach-In-Modus zu sehen. Im Teach-In-Modus ist der Kupplungshebel 55 hochgeklappt und der Motor 62 aus dem Orthesengelenk 4a ausgekuppelt. In diesem Zustand der Kupplungseinheit kann das Orthesengelenk 4a manuell, das heißt durch Muskel- oder Eigenkraft, bewegt werden. Im eingekuppelten Zustand dagegen sperrt das Schneckenradgetriebe jede Bewegung aus Richtung der Abtriebswelle 54 und lässt lediglich Bewegungen aus Richtung des Motors 62 zu.

Die Kupplungseinheit enthält den Kupplungshebel 55, welcher sich um eine Achse 74 zum Öffnen und Verschließen drehen lässt. Weiter ist die Trägerscheibe 64a zu sehen, sowie das Schneckenrad 60 und der obere Lagerblock 75. Im linken Bildbereich von Figur 5 b) ist über der Grundplatte 53 die Synchronscheibe 59b zu sehen, welche mit der Schneckenwelle 63 fest gekoppelt ist. Auf der der Grundplatte 53 abgewandten Seite der Schiene 41a befindet sich das Verschlusselement 36, welches die Abtriebswelle 54 in Achsrichtung der Abtriebswelle 54 sichert.

Figur 5 c) zeigt nun die Antriebseinheit 5a aus Figur 5 a) und b) in einer Rückansicht. Im Vordergrund und im mittleren Bildbereich ist der Motor 62 zu sehen. Im unteren Bildbereich ist die Grundplatte 53 sowie die Schienen 42 und 41a und das Verschlusselement 36, im oberen Bildbereich sind übereinander angeordnet die Halterung 65 des oberen Lagerblocks 75, die Platine 73, das Potentiometer 6 sowie der Kupplungshebel 55 zu sehen.

Figur 5 d) zeigt die Antriebseinheit 5a aus den Figuren 5 a) bis c) in einer perspektivischen, seitlichen Rückansicht mit der Schiene 41a und der Schiene 42, wobei der Motor 62 und die Platine 73 im Vordergrund zu sehen sind.

Figur 5 e) zeigt die Antriebseinheit 5a in einer perspektivischen Unteransicht, wobei im Vordergrund der Zahnriementrieb 59 mit den Synchronscheiben 59a und 59b zu sehen ist. In dieser Unteransicht ist die Verbindung zwischen der Grundplatte 53 und der Schiene 41a sowie der Schiene 42 im Drehpunkt 67 des Orthesengelenks 4a zu sehen. Die beiden Schienen 41a und 42 sind durch die Abtriebswelle 54 miteinander verbunden, die auf der der Schiene 42 abgewandten Seite der Schiene 41a ein Verschlusselement 37 aufweist. Im hinteren, rechteckigen Bereich der Grundplatte 53 führt die Schiene 41a vom Drehpunkt 67 des Orthesengelenks 4a weg. Die Grundplatte 53 und die Schiene 42 sind zueinander drehbar gelagert. Im mittleren Bereich der Grundplatte 53 befinden sich Schrauben 76a und 76b, welche zur Befestigung der Fest-Los-Lagerblöcke 61a und 61b an der Grundplatte 53 in die Durchgangsöffnungen 57a und 57b eingelassen und in die Fest-Los-Lagerblöcke 61a und 61b eingeschraubt sind (vgl. Figur 4 a) bis 4d)). Dabei verschwinden die Köpfe der Schrauben 76a und 76b in den Durchgangsöffnungen 57a und 57b, so dass diese der Unterseite der Grundplatte 53 bündig abschließen, und stützen sich an den Verjüngungen der Durchgangsöffnungen 57a und 57b ab. Zusätzlich sind Schrauben 76c benachbart zu den Schrauben 76a im hinteren Bereich der Grundplatte 53 zur Befestigung des Motorflansches 62a auf der Grundplatte 53 in die Durchgangsöffnungen 57c (vgl. Figur 4 a) bis c)) eingelassen und in die Grundplatte 53 eingeschraubt. Die Köpfe der Schrauben 76c verschwinden dabei in den Durchgangsöffnungen 57c, so dass diese mit der Unterseite der Grundplatte 53 bündig abschließen, und stützen sich an den Verjüngungen der Durchgangsöffnungen 57c ab. Im vorderen, halbkreisförmigen Bereich der Grundplatte 53 befindet sich eine Öffnung, durch die die Abtriebswelle 54 bis zum Orthesengelenk 4a hindurchführt. Die Schiene 42 führt über den vorderen, halbkreisförmigen Bereich der Grundplatte 53 vom Drehpunkt 67 des Orthesengelenks 4a weg.

Figur 5 f) zeigt die Antriebseinheit 5a aus den Figuren 5 a) bis e) in einer Seitenansicht, wobei nun die dem Zahnriementrieb 59 entgegengesetzte Seite der Antriebseinheit 5a im Vordergrund zu sehen ist. Im hinteren, rechteckigen Bereich der Grundplatte 53 sind der Motorflansch 62a, in welchem der Motor 62 gelagert ist, und der Lagerblock 61b zu sehen, in welchem die Schneckenwelle 63 durch ein Rillenkugellager drehbar gelagert ist.

Figur 5 g) zeigt die Antriebseinheit 5a in einer Draufsicht, wobei die Antriebseinheit 5a auf die gleiche Art ausgerichtet ist wie in Figur 5 f). Im Vordergrund ist die Platine 73 zu erkennen sowie die Schrauben 68 zur Befestigung der Halterung 65 des oberen Lagerblocks 75 auf den Fest-Los-Lagerblöcken 61a und 61b.

Figur 5 h) zeigt die Antriebseinheit 5a aus den Figuren 5 a) bis f) in einer weiteren Seitenansicht, wobei nun der Zahnriementrieb 59 mit den zueinander drehbar angeordneten Synchronscheiben 59a und 59b im Vordergrund im hinteren Bereich der Antriebseinheit 5a zu sehen ist.

Figuren 6 a) bis e) zeigen eine Orthese mit einem Schneckenantrieb, bei dem die Drehachse des Motors 93 und der Schneckenwelle 92 identisch sind.

Figur 6 a) zeigt eine vierteilige Orthese mit einem ersten Halteteil 2, einem zweiten Halteteil 3, einem dritten Halteteil 7 und einem vierten Halteteil 8. Das erste Halteteil 2 ist zur Aufnahme eines Unterschenkels ausgebildet, das zweite Halteteil 3 ist als Ringfassung zur Aufnahme eines Fußes ausgebildet, das dritte Halteteil 7 ist zur Aufnahme eines Oberschenkels ausgebildet und das vierte Halteteil 8 ist zur Aufnahme eines Rumpfes ausgebildet. Das erste und das zweite Halteteil 2 und 3 sind über ein linkes und ein rechtes Orthesengelenk 4a und 4a' zueinander drehbar um die Drehachse der Orthesengelenke 4a und 4a' verbunden. Die Drehachsen der Orthesengelenke 4a und 4a' sind in etwa identisch. Das erste und das dritte Halteelement 2 und 7 sind über ein linkes und ein rechtes Orthesengelenk 4b und 4b' zueinander drehbar um die Drehachsen der Orthesengelenke 4b und 4b' verbunden. Die Drehachsen der Orthesengelenke 4b und 4b' sind in etwa identisch. Das dritte und das vierte Halteelement 7 und 8 sind über ein linkes und ein rechtes Orthesengelenk 4c und 4c' zueinander drehbar um die Drehachsen der Orthesengelenke 4c und 4c' (in Figur 6 a) nicht dargestellt) verbunden. Die Drehachsen der Orthesengelenke 4c und 4c' sind in etwa identisch. Ferner ist am linken Orthesengelenk 4a eine Antriebseinheit 9a, am rechten Orthesengelenk 4b' eine Antriebseinheit 9b' und am linken Orthesengelenk 4c eine Antriebseinheit 9c angeordnet. Die dargestellten Antriebseinheiten weisen Schneckenantriebe auf.

Wie schon in den Figuren 1 und 2 weisen die Orthesengelenke in Figur 6 a) jeweils zwei Schienen auf, die in den Drehpunkten der Orthesengelenke drehbar zueinander verbunden sind. In Figur 6 a) ist es jedoch so, dass die Schienen 41 und 41', die jeweils Teile der Orthesengelenke 4a und 4a' sind, gleichzeitig jeweils Teile der Orthesengelenke 4b und 4b' sind. Die Schienen 42 und 42' verlaufen also jeweils durchgängig von den Drehpunkten der Orthesengelenke 4a und 4a' zu den Drehpunkten der Orthesengelenke 4b und 4b'. Gleichfalls sind die Schienen 43 und 43', welche jeweils Teile der Orthesengelenke 4b und 4b' sind, Teile der Orthesengelenke 4c und 4c'.

Wird die Orthese aus Figur 6 a) an einen menschlichen Körper angelegt, so umschließt das Halteteil 8 den Rumpf und das Halteteil 7 den linken Oberschenkel. Das Orthesengelenk 4c befindet sich dann im Bereich des linken Hüftgelenks, die Orthesengelenke 4b und 4b' jeweils in den Bereichen links und rechts des Kniegelenks. Das Halteteil 2 umschließt nach dem Anlegen der Orthese den linken Unterschenkel, das Halteteil 3 den linken Fuß, sodass sich die Orthesengelenke 4a und 4a' in den Bereichen links und rechts des Sprunggelenks befinden.

Figur 6 b) zeigt das Orthesengelenk 4b' der Orthese aus Figur 6 a). An dem Orthesengelenk 4b' ist die Antriebseinheit 9b' angeordnet, welche mittels eines Befestigungssystems 12 an der Schiene 43' befestigt ist. Die Antriebseinheit 9b' weist einen Motor 93 mit einem Gehäuse, eine Schneckenwelle 92 und ein Schneckenrad 91 auf. Das Gehäuse des Motors 92 ist mit dem Befestigungssystem 12 verbunden. Auf der dem Drehpunkt des Orthesengelenks 4b' zugewandten Seite des Motors 93 ist die Schneckenwelle 92 angeordnet. Die Schneckenwelle 92 befindet sich in formschlüssigem Kontakt mit dem Schneckenrad 91, welches im Drehpunkt des Orthesengelenks 4b' drehbar um die Drehachse des Orthesengelenks 4b' gelagert ist. Der Motor 93 führt eine Drehbewegung um eine Achse aus, die in etwa parallel zur Schiene 43 liegt. Diese Drehbewegung überträgt der Motor 93 auf die Schneckenwelle 92, deren Drehachse mit der Drehachse des Motors 93 identisch ist. Die Schneckenwelle 92 überträgt die Drehbewegung auf das Schneckenrad 91, dessen Drehbewegung eine Bewegung der Schienen 43' und 41' zueinander und damit des Orthesengelenks 4b' bewirkt.

Figur 6 c) zeigt eine vierteilige Orthese in einer schematischen Seitenansicht. Im Gegensatz zu Figur 6 a) ist hier lediglich das zweite Halteteil 3 dargestellt. Ferner ist zusätzlich zu den in Figur 6 a) auf der linken Seite der Orthese dargestellten Antriebseinheiten 9a und 9c eine Antriebseinheit 9b für das linke Orthesengelenk 4b vorhanden. Die Funktionsweise der Schneckenantriebe in den Antriebseinheiten 9a, 9b und 9c ist analog zur Funktionsweise der Antriebseinheit 9b', die unter Figur 6 b) beschrieben ist.

Figur 6 d) zeigt einen Regelkreis zum Betrieb einer Anordnung nach einer ersten Variante, die den Schneckenantrieb aus Figur 6 b) und eine Steuereinheit mit einem Hall-Sensor umfasst. Die Steuereinheit enthält einen Mikro-Controller (MC), einen Treiber und einen Hall-Sensor. Die Antriebseinheit in Figur 6 d) weist einen Motor, ein Getriebe (Getr), eine Schneckenwelle und ein Abtriebsrad bzw. Schneckenrad auf. Der Treiber versorgt den Motor 93 mit Strom, sodass dieser eine Drehbewegung ausführen kann. Die Drehbewegung des Motors 93 wird über das Getriebe (Getr) an die Schneckenwelle übertragen, welche ihrerseits die Drehbewegung an das Abtriebsrad bzw. Schneckenrad überträgt. Die Drehbewegung des Abtriebsrads bewirkt die Bewegung des zugehörigen Orthesengelenks. Der Hall-Sensor der Steuereinheit misst die Position des Motors, welche mit einem Sollwert verglichen wird. Die Position des Motors hängt dabei mit der Position des Orthesengelenks zusammen. Je nachdem wie der Vergleich der aktuellen Position des Motors mit dem Sollwert ausfällt, wird die Bewegung des Orthesengelenks fortgesetzt oder gestoppt. Anders ausgedrückt, durch den Sollwert wird ein elektronischer Anschlagswert des Orthesengelenks festgelegt. Die Geschwindigkeit der Bewegung des Orthesengelenks ist über die Stromversorgung des Motors regelbar. Figur 6 e) zeigt einen Regelkreis zur Veranschaulichung der Arbeitsweise einer Anordnung nach einer zweiten Variante, die den Schneckenantrieb aus Figur 6 b) und eine Steuereinheit mit einem Potentiometer umfasst. Die Steuereinheit enthält einen Mikro-Controller (MC), einen Treiber, ein Potentiometer (Poti) und einen Analog-Digital-Wandler. Die Antriebseinheit umfasst einen Motor, ein Getriebe (Getr) eine Schneckenwelle und ein Abtriebsrad bzw. Schneckenrad. Die Position des Orthesengelenks wird dieses Mal direkt mit dem Potentiometer analog gemessen und das Signal mittels eines Analog-DigitalWandlers in ein digitales Signal umgewandelt und an den Mikro-Controller (MC) gesendet. Die Position des Orthesengelenks wird wieder mit einem Sollwert verglichen. Je nachdem wie dieser Vergleich ausfällt, wird die Bewegung des Orthesengelenks fortgesetzt oder gestoppt. Auch hier bestimmt der Sollwert den elektronischen Anschlag des Orthesengelenks. Die Geschwindigkeit der Bewegung des Orthesengelenks ist über die Stromversorgung des Motors regelbar.

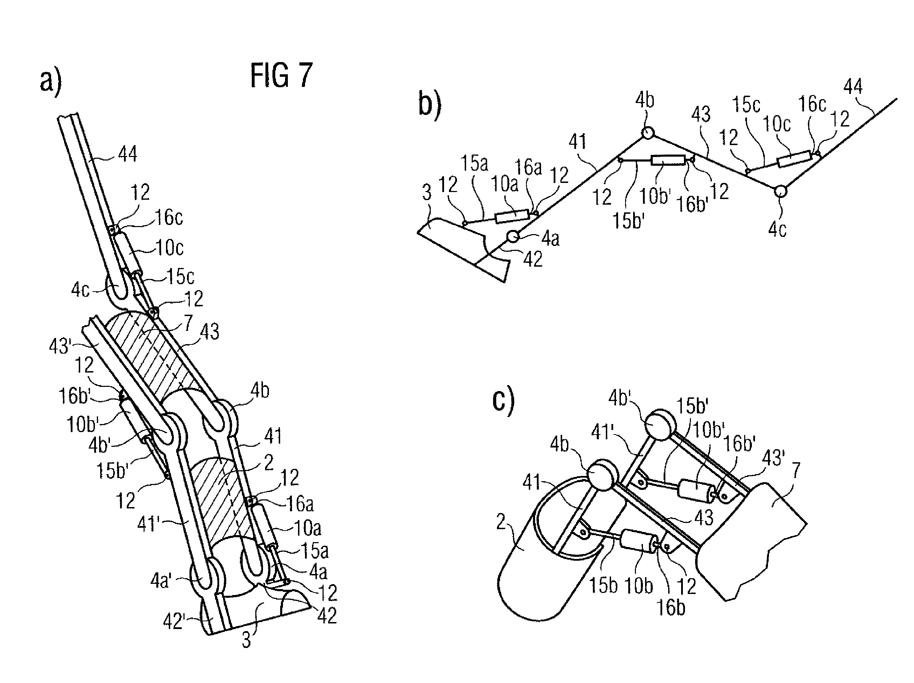

Figuren 7 a) bis c) zeigen eine Orthese mit Pneumatikzylindern, die als Antrieb für die Orthesengelenke dienen.

Figur 7 a) zeigt eine vierteilige Orthese. Wie in Figur 6 a) weist die Orthese in Figur 7 a) ein zweites Halteteil 3, welches als Ringfassung zur Aufnahme eines Fußes ausgebildet ist, ein erstes Halteteil 2 zur Aufnahme eines Unterschenkels, ein drittes Halteteil 7 zur Aufnahme eines Oberschenkels sowie ein viertes Halteteil 8 (in Figur 7 a) nicht dargestellt) zur Befestigung am Oberkörper auf. Die Anordnung der Orthesengelenke mit den dazugehörigen Schienen ist identisch zu Figur 6 a). Die Orthese weist ferner eine Antriebseinheit 10a auf, welche mittels eines Befestigungssystems 12 sowohl an der Schiene 42 als auch an der Schiene 41 befestigt ist. Im Bereich des Orthesengelenks 4b' weist die Orthese eine Antriebseinheit 10b' auf, die mit dem gleichen Befestigungssystem 12 sowohl an der Schiene 41 als auch an der Schiene 42 befestigt ist. Die Orthese weist eine dritte Antriebseinheit 10c im Bereich des Orthesengelenks 4c auf, die mit dem gleichen Befestigungssystem 12 sowohl an der Schiene 43 als auch an der Schiene 42 befestigt ist.

Im Folgenden wird der Aufbau einer Antriebseinheit am Beispiel der Antriebseinheit 10b' erklärt. Die übrigen Antriebseinheiten sind völlig analog aufgebaut.

Die Antriebseinheit 10b' weist einen Pneumatikzylinder auf, dessen zylinderförmiges Gehäuse mit einer Haltestange 16b' verbunden ist. An ihrem der Schiene 43' zugewandten Ende ist die Haltestange 16b' über das Befestigungssystem 12 an der Schiene 43' befestigt. In dem zylinderförmigen Gehäuse läuft ein Kolben, der mit der Gehäusewand eine Luftkammer einschließt. Weiter befindet sich in dem Gehäuse auf der der Luftkammer abgewandten Seite des Kolbens eine Schraubenfeder, die auf den Kolben einen Druck derart ausübt, dass die Luftkammer durch den Kolben verkleinert wird. Der Kolben ist mit einer Kolbenstange 15b' verbunden, die aus dem Gehäuse herausragt und an ihrem der Schiene 41' zugewandten Ende über das Befestigungssystem 12 an der Schiene 41' befestigt ist. Wird nun beispielsweise mittels eines Kompressors Luft in die Luftkammer gepumpt, dann wird der Kolben durch den Luftdruck gegen die Kraft der Feder in Richtung der Schiene 41' gedrückt. Dadurch schiebt sich die Kolbenstange 15b' weiter aus dem Gehäuse heraus. Wird die Luft aus der Luftkammer wieder abgelassen, dann sinkt der Luftdruck in der Luftkammer und der Kolben wird durch den Druck der Schraubenfeder wieder in Richtung der Schiene 43' geschoben, wodurch sich auch die Kolbenstange 15b' wieder in das Gehäuse hineinschiebt. Die Haltestange 16b' und die Kolbenstange 15b' üben dadurch ihrerseits eine Zug-oder Schubkraft auf die Schienen 41' und 43' auf und bewegen so das Orthesengelenk 4b'.

Figur 7 b) zeigt eine Seitenansicht der Orthese aus Figur 7 a). Ferner ist eine schematische Darstellung der Antriebseinheiten 10a, 10b' und 10c sowie Ihrer Befestigung an den Schienen der Orthese zu sehen. Wird die Orthese mit den Antriebseinheiten 10a, 10b' und 10c betrieben, bewegen sich die Kolbenstangen 15a, 15b' und 15c in die zylinderförmigen Gehäuse der Antriebseinheiten 10a, 10b' und 10c hinein bzw. heraus. Dadurch wird der Winkel, den zwei Schienen eines Orthesengelenks einschließen, an denen die Antriebseinheiten befestigt sind, verkleinert bzw. vergrößert und somit das zugehörige Orthesengelenk bewegt.

Figur 7 c) zeigt die Orthese aus Figur 7 a) und b) mit einer zusätzlichen Antriebseinheit 10b im Bereich des Orthesengelenks 4b in einer Draufsicht. Hier sind jedoch nur die Halteteile 2 und 7, sowie die Orthesengelenke 4b und 4b' im Bereich des Kniegelenks und die dazugehörigen Antriebseinheiten 10b und 10b' dargestellt. Die zusätzliche Antriebseinheit 10b ist analog zu den in Figur 7 a) dargestellten Antriebseinheiten 10a, 10b' und 10c aufgebaut und an der Orthese befestigt.

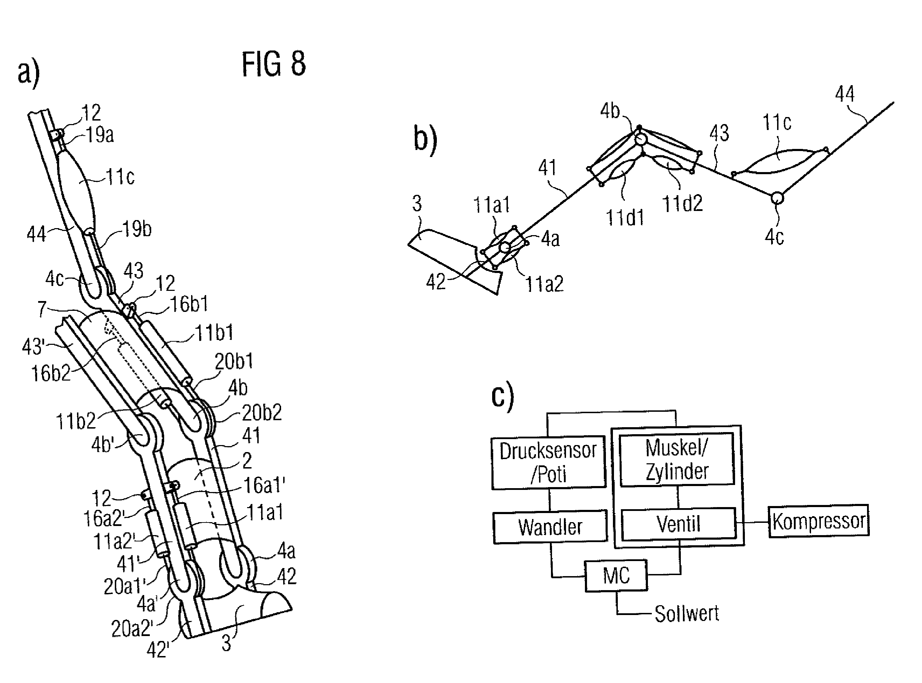

Figuren 8 a) bis c) zeigen eine Orthese, bei der die Orthesengelenke mit pneumatischen Muskeln und translatorischen Aktuatoren angetrieben werden.

Figur 8 a) zeigt eine vierteilige Orthese mit pneumatischen Muskeln und translatorischen Aktuatoren, die die Orthesengelenke antreiben. Wie schon in den Figuren 6 a) und 7 a) weist die Orthese ein erstes Halteteil 2 zur Aufnahme eines Unterschenkels, ein zweites Halteteil 3 zur Aufnahme eines Fußes, ein drittes Halteteil 7 zur Aufnahme eines Oberschenkels und ein viertes Halteteil (in Figur 8 a) nicht dargestellt) zur Aufnahme eines Oberkörpers auf. Das vierte Halteteil (nicht dargestellt) weist links und rechts Schienen 44 und 44' (nicht dargestellt) auf und ist über das linke und das rechte Orthesengelenk 4c bzw. 4c' (nicht dargestellt) mit dem dritten Halteteil 7 verbunden, welches links und rechts Schienen 43 und 43' aufweist. Das dritte Halteteil 7 ist über das linke und das rechte Orthesengelenk 4b und 4b' mit dem ersten Halteteil 2 verbunden, welches links und rechts Schienen 41 und 41' aufweist. Das erste Halteteil 2 ist über das linke und das rechte Orthesengelenk 4a und 4a' mit dem zweiten Halteteil 3 verbunden, welches links und rechts Schienen 42 und 42' aufweist. Das Orthesengelenk 4c wird mittels eines pneumatischen Muskels 11c bewegt, dessen dem Oberkörper zugewandtes oberes Ende mit einer Antriebsstange 19a verbunden ist. Die Antriebsstange 19a ist wiederum mittels eines Befestigungssystems 12 an der Schiene 44 befestigt. Die Orthesengelenke 4c und 4c' werden mittels eines pneumatischen Muskels 11c bewegt. Die Orthesengelenke 4a, 4a' und 4b, 4b' werden dagegen mittels translatorischen Aktuatoren bewegt.

Das dem Oberschenkel zugewandte untere Ende des pneumatischen Muskels 11c ist mit der Antriebsstange 19b verbunden und an der Schiene 43 befestigt. Wird nun Luft beispielsweise mittels eines Kompressors in den pneumatischen Muskel 11c gepumpt, dann nimmt dieser im Querschnitt zu und verkürzt sich dabei. Dadurch übt der pneumatische Muskel 11c eine Zugkraft auf die Schienen 43 und 44 auf, wodurch diese bzw. das vierte und das dritte Halteteil 7 zueinander hingezogen werden und sich der Winkel zwischen den Schienen 43 und 44 verkleinert. Das Bein wird zum Oberkörper hingezogen. Durch ein definiertes Ablassen der Luft aus dem pneumatischen Muskel 11c verlängert sich der pneumatische Muskel 11c wieder. Das Bein wird bewegt sich aufgrund seines Eigengewichtes nach unten. Dadurch vergrößert sich wieder der Winkel zischen den Schienen 43 und 44.

Die Orthesengelenke 4b, 4b' und 4a, 4a' werden mit translatorischen Aktuatoren 11b1 und 11b2 bzw. 11a1' und 11a2' bewegt. Die Aktuatoren 11b1 und 11b2 weisen zylinderförmige Gehäuse auf, die an ihren dem Orthesengelenk 4c zugewandten Enden mit Haltestangen 16b1 und 16b2 verbunden sind. Die Haltestangen 16b1 und 16b2 sind mittels Befestigungssystemen 12 an der Schiene 43 befestigt. An ihren dem Orthesengelenk 4b zugewandten Enden sind die Aktuatoren 11b1 und 11b2 über einen Seilzug mit einem Drahtseil 20b1 und einer Umlenkrolle 20b2, die am Orthesengelenk 4b angeordnet ist, miteinander verbunden. Die Aktuatoren 11a1' und 11a2' weisen ebenfalls zylinderförmige Gehäuse auf, die an ihren dem Orthesengelenk 4b zugewandten Enden mit Haltestangen 16a1' und 16a2' verbunden sind. Die Haltestangen 16a1' und 16a2' sind mittels Befestigungssystemen 12 an der Schiene 41 befestigt. An ihren dem Orthesengelenk 4a zugewandten Enden sind die Aktuatoren 11a1' und 11a2' über einen Seilzug mit einem Drahtseil 20a1' und einer Umlenkrolle 20a2', die am Orthesengelenk 4b angeordnet ist und über die das Drahtseil 20a1' läuft, miteinander verbunden.

Figur 8 b) zeigt eine weitere Variante einer vierteiligen Orthese, die analog zu Figur 8 a) aufgebaut ist, jedoch einen pneumatischen Muskel 11c zur Bewegung des Orthesengelenks 4c, pneumatischen Muskeln 11d1 und 11d2 zur Bewegung des Orthesengelenks 4b und translatorische Aktuatoren 11a1 und 11a2 zur Bewegung des Orthesengelenks 4a verwendet.

Figur 8 c) zeigt einen Regelkreis, der die Wirkungsweise des Pneumatikzylinders aus den Figuren 7 a) bis c) bzw. des pneumatischen Muskels aus den Figuren 8 a) und b) und einer Steuereinheit verdeutlicht. Die Steuereinheit enthält einen Mikrocontroller (MC), einen Drucksensor/Potentiometer (Poti) und einen Analog-Digital-Wandler. Die Antriebseinheit enthält einen Pneumatikzylinder bzw. einen pneumatischen Muskel, ein Ventil und einen angeschlossenen Kompressor. Hier wird die Position des Orthesengelenks direkt über das Potentiometer oder der Luftdruck im Pneumatikzylinder bzw. im pneumatischen Muskel über den Drucksensor gemessen, das gemessene analoge Signal mit dem Analog-Digital-Wandler in ein Digitalsignal umgewandelt und an den Mikro-Controller (MC) weitergeleitet. Dieser Wert (Istwert) für die Position des Orthesengelenks bzw. den Luftdruck im Pneumatikzylinder bzw. im pneumatischen Muskel wird mit einem Sollwert verglichen. Je nachdem wie dieser Vergleich zwischen Ist- und Sollwert ausfällt, wird der Kompressor gestartet, der Luft in den Pneumatikzylinder bzw. in den pneumatischen Muskel über das Ventil hineinpumpt, wodurch die Kolbenstange aus dem zylindrischen Gehäuse des Pneumatikzylinders herausgeschoben wird bzw. sich der Muskel im Querschnitt vergrößert und sich dadurch verkürzt, oder wird Luft aus dem Pneumatikzylinder bzw. dem pneumatischen Muskel über das Ventil herausgelassen, wodurch die Kolbenstange wieder in das Gehäuse des Pneumatikzylinders hineingeschoben wird bzw. sich der der Muskel wieder verlängert. Der Sollwert gibt auch hier wieder den elektronischen Anschlag des Orthesengelenks vor.

Die Geschwindigkeit der Bewegung eines Orthesengelenks, das mittels eines Pneumatikzylinders bewegt wird ist auf zwei Arten regelbar. Bei der ersten Variante wird die Verfahrgeschwindigkeit eines Pneumatikzylinders durch die Luftmenge eingestellt. Dabei kann die hineinströmende Zuluft (Zuluftdrosselung) oder die herausströmende Abluft (Abluftdrosselung) gedrosselt werden. Für die Drosselung der Luftmenge wird üblicherweise ein Drosselrückschlagventil eingesetzt. Die Geschwindigkeit kann bei diesen Zylindern über die bekannten, vom Hersteller definierten Eigenschaften gesteuert werden. Bei der zweiten Variante wird der Verfahrwinkel des Orthesengelenks stetig über eine gemessene Zeit mit einem Potentiometer gemessen. Die daraus resultierende Änderung des Verfahrwinkels pro Zeit wird mit einer vorgegebenen Sollgeschwindigkeit verglichen und die Luftzufuhr entsprechend geregelt.

Bei dem pneumatischen Muskel wird die Geschwindigkeit der Bewegung des Orthesengelenks ebenfalls über die Luftzufuhr geregelt. Hierbei werden nichtlineare Regelungskonzepte verfolgt, da die Muskelkraft sowie das Muskelvolumen nichtlinear von der Kontraktionslänge und dem Innendruck abhängen.

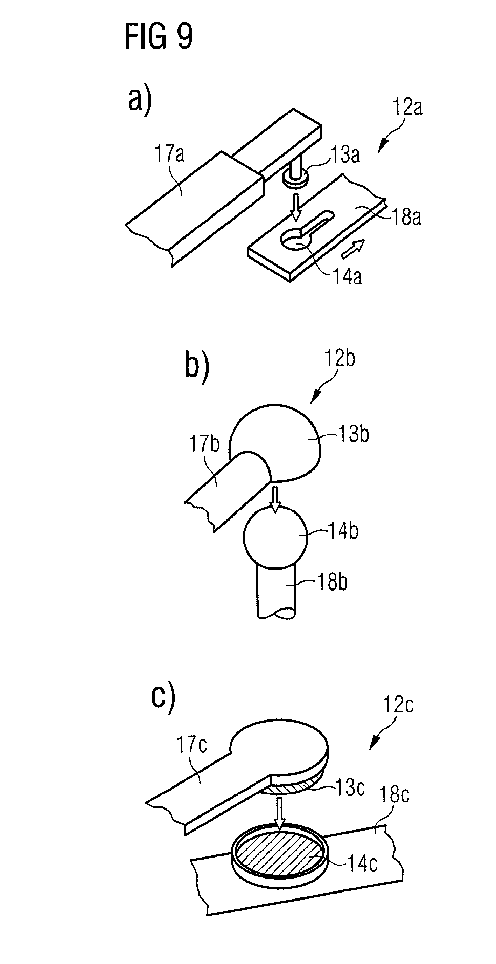

Figur 9 zeigt verschiedene Befestigungssysteme, die als Befestigungssystem 12 in den Figuren 6 b), 7 a) bis c) und 8 a) zur Befestigung der Antriebseinheiten an den Schienen der Orthesengelenke geeignet sind.

Die Figur 9 a) zeigt einen Bajonettverschluss 12a. Dieser verbindet zwei Teile 17a und 18a miteinander, wobei das erste Teil 17a mit einer Antriebseinheit verbunden ist, wie z.B. mit dem der Schiene 43' zugewandten Ende der Antriebsstange 16b' der Antriebseinheit 10b' in Figur 7 a). Das zweite Teil 18a ist mit einer Schiene verbunden bzw. in der Schiene enthalten oder an die Schiene montiert, an der eine Antriebseinheit befestigt werden soll, wie z.B. die Schiene 43' der Figur 7 a). Das erste Teil 17a weist einen Haken 13a und das zweite Teil eine Aufnahme 14a mit einer Verjüngung auf. Der Haken 13a wird senkrecht in die Aufnahme 14a gesteckt und in die Verjüngung hinein geschoben, so dass der Haken 13a am Herausrutschen in Richtung des zweiten Teils und senkrecht aus der Aufnahme 14a heraus gehindert wird.

Figur 9 b) zeigt ein Kugelkopfverschlusssystem 12b, welcher alternativ zu einem Bajonettverschluss 12a zur Befestigung einer Antriebseinheit an einer Schiene verwendet werden kann. Das Kugelkopfsystem 12b verbindet zwei Teile 17b und 18b miteinander, wobei das erste Teil 17b ein pfannenförmiges Klemmelement 13b und das zweite Teil 18b einen kugelförmigen Kopf 14b aufweist. Der kugelförmige Kopf 14b wird nun in das pfannenförmige Klemmelement 13b hineingedrückt, so dass sich der kugelförmige Kopf 14b mit der Hinterschneidung des pfannenförmigen Klemmelementes 13b verklemmt.

Figur 9 c) zeigt ein Magnetstecksystem 12c, welches alternativ zum Bajonettverschluss 12a und zum Kugelkopfsystem 12b zur Befestigung einer Antriebseinheit an einer Schiene eines Orthesengelenks verwendet werden kann. Das Magnetstecksystem 12c verbindet zwei Teile 17c und 18c miteinander, wobei das erste Teil 17c einen magnetischen zylindrischen Stift und das zweite Teil 18c eine magnetische ringförmige Aufnahme aufweist. Nun wird der magnetische zylindrische Stift 13c in die magnetische ringförmige Aufnahme 14c hineingesteckt, wodurch sich eine magnetische Verbindung zwischen den Teilen 17c und 18c ergibt.

Figur 10 a) bis h) zeigt die Kupplungseinheit der Antriebseinheit 5a aus den Figuren 5 a) bis h), welche beim Ein- und Ausschalten des Teach-In-Modus betätigt wird, in geöffnetem, d.h. ausgekuppeltem, Zustand.

Figur 10 a) zeigt die Kupplungseinheit aus den Figu-ren 5 a) bis h) in einer perspektivischen, seitlichen Vorderansicht. Die Kupplungseinheit enthält eine Halterung 65 für den oberen Lagerblock 75, die mit dem oberen Lagerblock 75 einstückig ausgebildet ist. Wie in den Figuren 5 a) bis h) ist der Kupplungshebel 55 am äußeren, seitlichen Rand der Schnittfläche zwischen dem oberen Lagerblock 75 und der Halterung 65 in zwei Punkten um eine Achse 74 drehbar angeordnet. Die zugehörige Drehachse 74 verläuft durch die angesprochene Schnittfläche und horizontal zur Grundplatte 53 (vgl. bspw. Figur 5 a). Der Kupplungshebel 55 ist doppel-S-förmig ausgebildet, wobei die parallelen oberen Enden durch einen horizontal zur Grundplatte 53 verlaufenden Quersteg miteinander verbunden sind und die parallelen unteren Enden an dem oberen Lagerblock 75 drehbar aufgehängt sind. Der Kupplungshebel 55 ist um die Drehachse 74 um einen Winkel zwischen 0° und über 90° drehbar. In dem oberen Lagerblock 75 unterhalb des Querstegs des Kupplungshebels 55 ist eine kreisscheibenförmige Aussparung eingebracht, die als Aufnahme 71 für ein Potentiometer 6 dient. In der Halterung 65 sind zum seitlichen Rand bzw. zu den kurzen Seiten der gedachten rechteckigen Grundplatte 53 (siehe bspw. Figur 5 a)) und senkrecht zur Grundplatte 53 verlaufende Durchgangsöffnungen 66a und 66b zur Aufnahme von Schrauben 68a und 68b angeordnet, die zur Befestigung der Halterung 65 auf den Fest-Los-Lagerblöcken 61a und 61b der Antriebseinheit 5a dienen. Die Durchgangsöffnungen 66a und 66b weisen zu den Fest-Los-Lagerblöcken 61a und 61b hin Verjüngungen auf, sodass die Köpfe der Schrauben 68a und 68b in die Durchgangsöffnungen 66a und 66b bis zu den Verjüngungen absenkbar sind und bündig mit der Oberseite der Halterung 65 abschließen. Auf der dem Quersteg des Kupplungshebels 55 abgewandten Unterseite des oberen Lagerblocks 75 befindet sich wie in den Figuren 5 a) bis h) die Trägerscheibe 64a und die Kupplungsscheibe 64b. Die Trägerscheibe 64a und die Kupplungsscheibe 64b sind fest miteinander verbunden und zusammen senkrecht zum oberen Lagerblock 75 auf der gedachten Abtriebswelle 54 (vgl. Figuren 5 a) bis h) ) verschiebbar. Ferner sind Trägerscheibe 64a und Kupplungsscheibe 64b fest mit der Abtriebswelle 54 gekoppelt, sodass Drehbewegungen der Kupplungsscheibe 64b über die Trägerscheibe 64a direkt auf die Abtriebswelle 54 übertragbar sind. Darunter wiederum befindet sich das Schneckenrad 60, dessen Position in einer Richtung senkrecht zum oberen Lagerblock 75 fixiert ist und welches mit der Abtriebswelle 54 nicht fest gekoppelt ist, sodass sowohl Drehbewegungen des Schneckenrads 60 nicht unmittelbar auf die Abtriebswelle 54 als auch Drehbewegungen der Abtriebswelle 54 nicht unmittelbar auf das Schneckenrad 60 übertragbar sind.

Figur 10 b) zeigt die Kupplungseinheit in einer Seitenansicht. Hier ist die Form des Kupplungshebels 55 zu erkennen. Dieser ist in etwa S-förmig geformt und um die Drehachse 74 drehbar an den seitlichen äußeren Rändern der Schnittfläche zwischen der Halterung 65 und des oberen Lagerblocks 75 gelagert. Im geöffneten Zustand ist der Kupplungshebel 55 mindestens um einen Winkel zwischen 45° und 90° nach oben um die Drehachse 74 gedreht. Im geschlossenen Zustand drückt der Kupplungshebel 55 mit seinem der Drehachse 74 benachbarten Bereich auf die Trägerscheibe 64a, an welcher die Kupplungsscheibe 64b befestigt ist. Die Kupplungsscheibe 64b weist einen Hauptkörper und einen Kopf (hier nicht dargestellt) auf, wobei der Hauptkörper im Gegensatz zur Trägerscheibe 64a nicht rund ausgebildet ist, sondern die Grundfläche eines Zwölfecks besitzt. Durch den Druck des Kupplungshebels 55 auf die Trägerscheibe 64a wird die Kupplungsscheibe 64b in das Schneckenrad 60 hineingedrückt, welches in seinem Inneren eine zwölfeckige Aussparung aufweist. Die Kupplungsscheibe 64b verbindet sich dadurch formschlüssig mit dem Schneckenrad 60.

Figur 10 c) zeigt die Kupplungseinheit in einer seitlichen Schnittansicht. Figur 10 d) zeigt die Kupplungseinheit in einer dazugehörigen Vorderansicht, wobei die Linie W-W die Schnittlinie für die Figur 10 c) darstellt.

In Figur 10 c) ist im oberen Bereich der geöffnete Kupplungshebel 55 zu sehen. Dieser ist in Richtung des oberen Lagerblocks 75 hin schließbar und in Richtung der Halterung 65 hin öffenbar. Auf der dem Kupplungshebel 55 abgewandten Seite des oberen Lagerblocks 75 ist die zum oberen Lagerblock 75 in senkrechter Richtung verschiebbare Trägerscheibe 64a angeordnet. Auf der dem oberen Lagerblock 75 abgewandten Seite der Trägerscheibe 64a ist die Kupplungsscheibe 64b angeordnet, die einen geringeren Durchmesser als die Trägerscheibe 64a aufweist. Die Kupplungsscheibe 64b weist einen Kopf 46 auf, der einen etwas größeren Durchmesser misst, als der Hauptkörper der Kupplungsscheibe 64b. Der Kopf 46 der Kupplungsscheibe 64b ist etwa zu 1/5 der Dicke der Kupplungsscheibe 64b in die Trägerscheibe 64a formschlüssig eingelassen. In einem Abstand vom Mittelpunkt der Trägerscheibe 64a, wobei die Drehachse des Orthesengelenks 4a durch diesen Mittelpunkt läuft, von in etwa dem halben Radius der Trägerscheibe 64a laufen zwei diametral angeordnete Schrauben 70 senkrecht und von der dem oberen Lagerblock 75 zugewandten Seite der Trägerscheibe 64a durch die Trägerscheibe 64a und vollständig durch die Kupplungsscheibe 64b, sodass die oberen Enden der Schrauben 70 bündig mit der dem oberen Lagerblock 75 zugewandten Seite der Trägerscheibe 64a und die unteren Enden der Schrauben 70 bündig mit der der Trägerscheibe 64a abgewandten Seite der Kupplungsscheibe 64b abschließen. Die zwei Schrauben 70 verbinden die Kupplungsscheibe 64b fest mit der Kupplungsscheibe 64a. Auf der der Trägerscheibe 64a abgewandten Seite der Kupplungsscheibe 64b ist das Schneckenrad 60 angeordnet. Dieses ist in einer Richtung senkrecht zum oberen Lagerblock 75 fixiert. Das Schneckenrad 60 weist in seinem der Kupplungsscheibe 64b zugewandten Bereich eine zwölfeckige Aussparung 77 auf, die den Hauptkörper der Kupplungsscheibe 64b vollständig und formschlüssig in sich aufnehmen kann. Ferner sind in einem Abstand vom Mittelpunkt der Aussparung 77, wobei die Drehachse des Orthesengelenks 4a durch diesen Mittelpunkt läuft, von in etwa dem halben Radius der Aussparung 77 vier Druckstössel 72 innerhalb der Aussparung 77 angeordnet. Die Funktion der Druckstössel 72 wird anhand von Figur 10 f) erläutert. In den mittleren Bereichen der Trägerscheibe 64a, der Kupplungsscheibe 64b und des Schneckenrads 60 sind Aussparungen 79a, 79b und 78 für die Abtriebswelle 54 eingelassen (vgl. Figuren 4 a) bis d) und 5 a) bis h)). Figur 10 d) zeigt eine Vorderansicht der Kupplungseinheit der Antriebseinheit 5a aus den Figuren 5 a) bis h) . Im oberen Bereich befindet sich der geöffnete Kupplungshebel 55, der in Richtung des oberen Lagerblocks 75 bzw. aus der Bildebene hinaus durch drehen um die Achse 74 schließbar ist und in Richtung der Halterung 65 bzw. in die Bildebene hinein durch drehen um die Achse 74 öffenbar ist. Auf der dem Kupplungshebel 55 abgewandten Seite des oberen Lagerblocks 75 ist die entlang einer Richtung senkrecht zum oberen Lagerblock 75 verschiebbare Trägerscheibe 64a angeordnet, an deren dem oberen Lagerblock 75 abgewandten Seite die Zwölf-Kant-Kupplungsscheibe 64b befestigt ist. Auf der der Trägerscheibe 64a abgewandten Seite der Kupplungsscheibe 64b ist das Schneckenrad 60 angeordnet, dessen Position in einer Richtung senkrecht zum oberen Lagerblock 75 fixiert ist.

Figur 10 e) zeigt eine Draufsicht auf die Kupplungseinheit aus Figur 10 a) bis d). Die Schnittlinie Y-Y zeigt die Schnittfläche für die Figur 10 f) an, welche die Kupplungseinheit in einer Schnittansicht zeigt. In Figur 10 e) verläuft die Schnittlinie Y-Y schräg über die Kupplungseinheit, das heißt nicht parallel zu den Seitenkanten der Halterung 65 oder des Kupplungshebels 55, von der Halterung 65 zum oberen Lagerblock 75. In der Halterung 65 sind im Bereich der Seitenkanten je zwei Durchgangsöffnungen 66a und 66b angeordnet, die Schrauben 68a und 68b (vgl. bspw. Figur 5 a)) zur Befestigung der Halterung 65 an den Fest-Los-Lagerblöcken 61a und 61b aufnehmen. Im mittleren Bildbereich befindet sich der Kupplungshebel 55. Auf der der Kupplungseinheit zugewandten Seite bzw. in die Bildebene hinein ist eine Aufnahme 71 für ein Potentiometer 6 (vgl. bspw. Figur 5 a)) angeordnet. Die Aufnahme 71 ist in den oberen Lagerblock 75 eingelassen.

Figur 10 f) zeigt die Kupplungseinheit in einer schrägen Schnittansicht entlang der Linie Y-Y, die in Figur 10 e) eingezeichnet ist. Auf der dem Kupplungshebel 55 abgewandten Seite des oberen Lagerblocks 75 ist die Trägerscheibe 64a angeordnet, an der die Kupplungsscheibe 64b mittels Schrauben 70 befestigt ist, die in dieser Schnittansicht nicht dargestellt sind. Die Kupplungsscheibe 64b ist dabei etwa zu 1/5 der Dicke der Kupplungsscheibe 64b in die Trägerscheibe 64a eingelassen. In einem Abstand vom Mittelpunkt der Trägerscheibe 64a von in etwa dem halben Radius der Trägerscheibe 64a sind in die Trägerscheibe 64a und zu dieser formschlüssig auch in die Kupplungsscheibe 64b vier zylinderförmige Aussparungen 51 eingebracht. Die Aussparungen 51 beginnen etwa nach 1/5 der Dicke der Trägerscheibe 64a von der dem oberen Lagerblock 75 zugewandten Seite der Trägerscheibe 64a her kommend in Richtung der Kupplungsscheibe 64b, führen zur Kupplungsscheibe 64b und durch diese komplett hindurch zum Schneckenrad 60 hin. Entlang bis zu etwa 1/4 der Dicke der Kupplungsscheibe 64b vom Schneckenrad 60 aus gesehen, sind die Aussparungen in der Kupplungsscheibe 64b verjüngt, sodass sich Hinterschneidungen 45 ergeben. In den Aussparungen 51 befinden sich Federn 69, welche auf Druck vorgespannt sind. Unterhalb der Federn in Richtung des Schneckenrads 60 sind Druckstössel 72 angeordnet, deren Köpfe den gleichen Radius wie die zylinderförmigen Aussparungen 51 aufweisen. Die Köpfe der Druckstössel 72 befinden sich innerhalb der Aussparungen 51 und werden durch den Druck der Federn 69 im hier dargestellten ausgekuppelten Zustand der Kupplungseinheit gegen die Hinterschneidungen 45 gedrückt. Die Rümpfe der Druckstössel befinden sich im hier dargestellten ausgekuppelten Zustand der Kupplungseinheit in der Aussparung 77 des Schneckenrads 60. Wird der Kupplungshebel 55 in Richtung des oberen Lagerblocks 75 um die Achse 74 gedreht, drückt der der Achse 74 benachbarte Bereich des Kupplungshebels 55 auf die Trägerscheibe 64a. Hierdurch wird die senkrecht zum oberen Lagerblock 75 verschiebbare Trägerscheibe 64a in Richtung des Schneckenrads 60 geschoben, wobei sich die Druckstössel 72 gegen die Kraft der Federn 69 weiter in die zylinderförmigen Aussparungen 51 hineinschieben und die Federn 69 zusammendrücken, bis sich auch die kompletten Rümpfe der Druckstössel 72 in den Aussparungen 51 befinden (vgl. Figur 11 f)). Der Hauptkörper der Kupplungsscheibe 64b, welcher der Kupplungsscheibe 64b ohne den Kopf 46 entspricht, befindet sich nun formschlüssig in der Aussparung 51 des Schneckenrads 60, was zu einer Kopplung zwischen der Trägerscheibe 64a und dem Schneckenrad 60 führt. Auf diese Weise kann die Antriebseinheit 5a aus den Figuren 5 a) bis h) in das Orthesengelenk 4a eingekuppelt werden. Die Abtriebswelle 54, dargestellt in den Figuren 4 a) bis d) und 5 a) bis h) , läuft durch die Aussparungen 78, 79a und 79b des Schneckenrads 60, der Kupplungsscheibe 64b und der Trägerscheibe 64a, dargestellt in Figur 10 f). Das Schneckenrad 60 überträgt die ursprünglich vom Motor 62 erzeugte Drehbewegung auf die eingekuppelte Kupplungsscheibe 64b, welche ihrerseits die Drehbewegung auf die Trägerscheibe 64a überträgt. Die Trägerscheibe 64a und die Kupplungsscheibe 64b sind über eine Passfeder-NutVerbindung formschlüssig mit der Abtriebswelle 54 verbunden, können jedoch in einer Richtung parallel zur Achse der Abtriebswelle 54 translatorisch verschoben werden. Die Abtriebswelle 54 führt damit ebenfalls die Drehbewegung der Trägerscheibe 64a aus. Die Abtriebswelle 54 überträgt die Drehbewegung wiederum auf die Schiene 42, wodurch sich das Orthesengelenk 4a bewegt (vgl. Figur 4 a) bis d)).

Figur 10 g) zeigt das Schneckenrad 60, die Trägerscheibe 64a und die Kupplungsscheibe 64b in einer Schnittansicht entlang der Linie O-O, welche in Figur 10 h) eingezeichnet ist. Diese Schnittansicht entspricht der Schnittansicht in Figur 10 c). In der Trägerscheibe 64a und der Kupplungsscheibe 64b sind die senkrecht verlaufenden Schrauben 70 zu sehen, die die Kupplungsscheibe 64b mit der Trägerscheibe 64a verbinden. Dabei ist der Kopf 46 der Kupplungsscheibe 64b, der eine Dicke von etwa 1/5 der Dicke der Kupplungsscheibe 64b aufweist, formschlüssig in die Trägerscheibe 64a eingelassen. Im mittleren Bereich des Schneckenrads 60, der Kupplungsscheibe 64b und der Trägerscheibe 64a befinden sich Aussparungen 78, 79b und 79a zur Aufnahme der Abtriebswelle 54 (vgl. Figur 5 a) bis h)). In dem der Kupplungsscheibe 64b zugewandten Bereich des Schneckenrads 60 ist die zwölfeckige Aussparung 77 zu sehen, welche die Kupplungsscheibe formschlüssig aufnehmen kann. In der Aussparung 77 sind Druckstössel 72 angeordnet, die sich in dem Verbund aus Kupplungsscheibe 64b und Trägerscheibe 64a vollständig versenken lassen (vgl. Figur 11 f)).

Figur 10 h) zeigt eine Vorderansicht des Schneckenrades 60, der Kupplungsscheibe 64b und der Trägerscheibe 64a. Die Linie O-O zeigt die Schnittfläche an, aus welcher sich die Schnittfigur 10 g) ergibt.

Figur 11 a) zeigt die Kupplungseinheit in einer perspektivischen, seitlichen Vorderansicht, wie in Figur 10 a), nun jedoch im geschlossenen bzw. eingekuppelten Zustand. Der Kupplungshebel 55 ist in seiner geschlossenen Position, das heißt um 0° um die Drehachse 74 verdreht, wobei der der Drehachse 74 benachbarte Bereich des Kupplungshebels 55 auf die Trägerscheibe 64a drückt, wodurch die Kupplungsscheibe 64b (hier nicht dargestellt) in das Schneckenrad 60 hineindrückt wird, dessen Position in einer Richtung senkrecht zum oberen Lagerblock 75 fixiert ist.

Figur 11 b) zeigt die Kupplungseinheit in einer Seitenansicht, wie in Figur 10 b), nun jedoch im geschlossenen bzw. eingekuppelten Zustand. Der Kupplungshebel 55 drückt mit seinem der Drehachse 74 benachbarten Bereich auf die Trägerscheibe 74, wobei die Kupplungsscheibe 64b (hier nicht dargestellt) in das Schneckenrad 60 hineingedrückt wird.

Figur 11 c) zeigt die Kupplungseinheit in einer Schnittansicht entlang der Linie P-P, welche in Figur 11 d) dargestellt ist, wie in Figur 10 c), nun jedoch im geschlossen bzw. eingekuppelten Zustand. Die Kupplungsscheibe 64b ist mittels der Schrauben 70 mit der Trägerscheibe 64a verbunden. Der Hauptkörper der Kupplungsscheibe 64b, welcher der Kupplungsscheibe 64b ohne den Kopf 46 entspricht, befindet sich vollständig im Schneckenrad 60. Dabei stützt sich der Kopf 46 der Kupplungsscheibe 64b auf dem der Trägerscheibe 64a zugewandten Rand der Aussparung 77 ab, sodass sich ein formschlüssiger Verbund zwischen Schneckenrad 60, Kupplungsscheibe 64b und Trägerscheibe 64a ergibt.

Figur 11 d) zeigt eine Vorderansicht der Kupplungseinheit, wie in Figur 10 d), nun jedoch im geschlossenen Zustand. Die Schnittlinie P-P zeigt die Schnittfläche für die Figur 11 c) an. Die Kupplungsscheibe 64a und das Schneckenrad 60 bilden einen formschlüssigen Verbund.

Figur 11 e) zeigt eine Draufsicht der Kupplungseinheit, wie in Figur 10 e), nun jedoch im geschlossenen Zustand. Die schräg von der Halterung 65 zum Kupplungshebel 55 verlaufende Schnittlinie S-S zeigt die Schnittfläche für die Figur 11 f) an. Im Vergleich zu Figur 10 e) befindet sich der Kupplungshebel 55 nun seiner geschlossenen Position, also um 0° um die Drehachse 74 verdreht, direkt über dem oberen Lagerblock 75 und verdeckt diese.

Figur 11 f) zeigt eine schräge Schnittansicht der Kupplungseinheit, wie in Figur 10 f), nun jedoch im geschlossenen Zustand. Der Kupplungshebel 55 befindet sich in seiner geschlossenen Position, sodass der der Drehachse 74 benachbarte Bereich des Kupplungshebels 55 einen Druck auf die dem oberen Lagerblock 75 zugewandten Seite der Trägerscheibe 64a ausübt. Durch den Druck des Kupplungshebels 55 auf die Trägerscheibe 64a, wird die Kupplungsscheibe 64b in die Aussparung 77 des Schneckenrads gedrückt. Dabei schieben sich die Druckstössel 72 gegen die Kraft der in den Aussparungen 51 gelagerten Federn 69 vollständig in die Aussparungen 51 hinein (vgl. Figur 10 f)). Die Federn 69, die auf Druck vorgespannt sind, werden dabei auf etwa die Hälfte ihrer vorgespannten Länge zusammengedrückt. Der Kopf 46 der Kupplungsscheibe 64b stützt sich auf dem der Trägerscheibe 64a zugewandten Seite des Schneckenrads ab. Ebenso stützt sich der äußere Rand der Trägerscheibe 64a, der die Kupplungsscheibe 64b im Durchmesser überragt, auf dem äußeren Rand um die Aussparung 77 des Schneckenrads 60 ab.

Figuren 11 g) bis j) zeigen nochmals das Schneckenrad 60, die Trägerscheibe 64a und die Kupplungsscheibe 64b in Detailansichten, wobei die Figuren 11 g) und 11 h) wie in den Figuren 10 g) und h) dargestellt sind, in geschlossenem bzw. eingekuppeltem Zustand.

Figur 11 g) zeigt eine Schnittansicht entlang der Linie I-I, welche in Figur 11 h) zu sehen ist. Die Darstellung des Schneckenrads 60, der Kupplungsscheibe 64b sowie der Trägerscheibe 64a entsprechen der Darstellung in Figur 11 c). Im eingekuppeltem Zustand bilden das Schneckenrad 60, die Kupplungsscheibe 64b und die Trägerscheibe 64a einen formschlüssigen Verbund, wobei sich der Hauptkörper der Kupplungsscheibe 64b in der Aussparung 77 des Schneckenrads 60 befindet. Der Kopf 46 der Kupplungsscheibe 64b ist in die Trägerscheibe 64a eingelassen und schließt bündig mit der Trägerscheibe 64a ab, sodass sich der Kopf 46 und die Trägerscheibe 64a auf dem die Aussparung 77 begrenzenden und zur Trägerscheibe 64a zugewandten Rand des Schneckenrads 60 abstützen.

Figur 11 h) zeigt eine Vorderansicht des Schneckenrades 60 und der Trägerscheibe 64a im eingekuppelten Zustand. Die Darstellung des Schneckenrads 60 und der Trägerscheibe 64a entspricht der Darstellung in Figur 10 h). Die Trägerscheibe 64a und das Schneckenrad 60 bilden einen formschlüssigen Verbund.

Figur 11 i) zeigt eine Schnittansicht entlang der Linie J-J die in Figur 11 h) dargestellt ist. Die Schnittfläche entlang der Linie J-J entspricht der Berührungsfläche zwischen der Trägerscheibe 64a und dem der Trägerscheibe 64a zugewandten Rand des Schneckenrads 60, welcher die zwölfeckige Aussparung 77 begrenzt. Im Inneren der Figur befindet sich die Aussparung 79b der Kupplungsscheibe 64b, durch welche im montierten Zustand die Abtriebswelle 54 führt (vgl. Figuren 5 a) bis h)). Im mittleren Bildbereich ist die zwölfeckige Schnittfläche des Hauptkörpers der Kupplungsscheibe 64b zu sehen. Im äußeren Bereich der Figur ist der Rand des Schneckenrads 60 zu sehen, der die zwölfeckige Aussparung 77 seitlich begrenzt. Im Abstand von in etwa des halben Radius vom Mittelpunkt des Schneckenrads 60, wobei die Drehachse des Orthesengelenks 4a durch diesen Mittelpunkt verläuft, sind die senkrecht zur Schnittfläche verlaufenden Aussparungen 51, in welchen sich die eingeschobenen Druckstössel 72 befinden, in der Kupplungsscheibe 64b quadratisch angeordnet. In etwa im gleichen Abstand vom Mittelpunkt des Schneckenrads sind zwischen jeweils zwei Aussparungen 51 die zwei senkrecht zur Schnittfläche verlaufenden Schrauben 72 diametral in der Kupplungsscheibe 64b angeordnet. Die Schrauben 72 verbinden die Trägerscheibe 64a (hier nicht dargestellt) und die Kupplungsscheibe 64b fest miteinander.

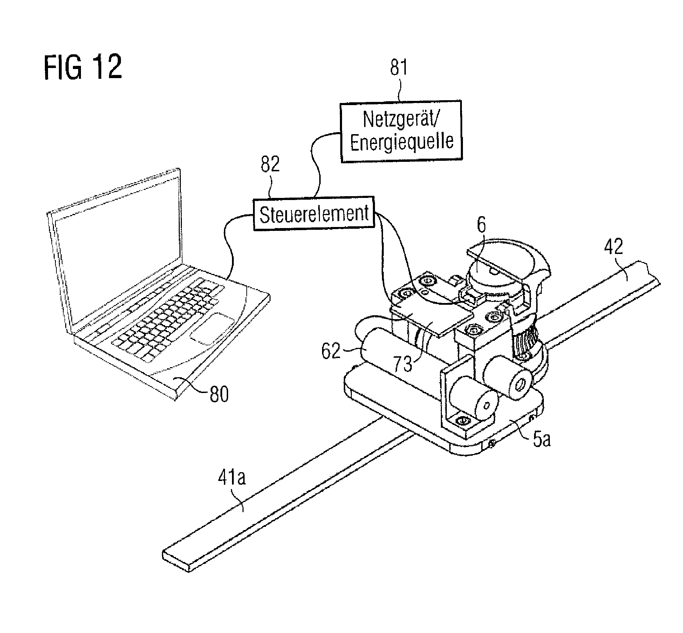

Figur 12 zeigt eine Anordnung einer Antriebseinheit 5a mit einer Schiene 41a und einer Schiene 42 (vgl. Figuren 5 a) bis h)) und einer Steuereinheit. Die Steuereinheit umfasst ein Bedienelement 80, ein Steuerelement 82 und eine Energiequelle 81. Die Antriebseinheit 5a weist eine Platine 73 auf, die die elektrischen Anschlüsse für die Komponenten der Antriebseinheit 5a, wie z.B. den Anschluss für den Motor 62, bündelt. Die Anschluss-Platine ist mit dem Steuerelement 82 elektrisch verbunden. Das Potentiometer 6, welches auf der Antriebseinheit 5a angeordnet ist, ist direkt mit dem Steuerelement 82 elektrisch verbunden. Das Steuerelement 82 ist an eine Energiequelle 81, wie z.B. ein Netzgerät oder einen Akkumulator, angeschlossen. Weiterhin ist das Steuerelement 82 mit einem Bedienelement 80, z.B. mit einem PC oder einem Laptop, verbunden. Diese Verbindung kann beispielsweise über ein USB-Kabel oder über ein Netzwerk hergestellt werden. Das Bedienelement verfügt über ein Speicherelement zum Speichern von Therapiedaten, welche mit einem Sensor, wie z.B. dem Potentiometer 6, gemessen wurden, und zum Speichern von Referenz- bzw. Sollwerten, die über das Bedienelement 80 eingegeben wurden. Die Therapiedaten schließen beispielsweise die Randwerte für den maximal möglichen Bewegungsbereich des entsprechenden Körpergelenks ein, während die Referenzwerte beispielsweise die vorgegebenen Randwerte für einen erlaubten Bewegungsbereich bzw. die Werte für die elektronischen Anschläge des entsprechenden Orthesengelenks sein können. Derartige Referenzwerte können auch Bestandteile von Therapieprogrammen sein, welche mithilfe des Bedienelements 80 programmiert oder über das Bedienelement 80 von einem externen Speichermedium, wie z.B. von einem USB-Stick übertragen wurden. Des Weiteren verfügt das Bedienelement 82 über einen Mikroprozessor bzw. Mikro-Controller, auf dem Therapieprogramme programmiert werden können und der Therapieprogramme ausführt. Das Steuerelement enthält einen Treiber zum Steuern des Motors 62 bzw. des gesamten Gelenkantriebs und einen Analog-Digital-Wandler zum Umwandeln von analogen Signalen in digitale Signale.

Bei einer Therapieeinheit wird eine Orthese wie in Figur 2 u.a. mit einer Antriebseinheit 5a in eingekuppeltem Zustand verwendet. Während der Bewegung des Orthesengelenks 4a (in Figur 12 nicht dargestellt), die von der Antriebseinheit 5a bewirkt wird, misst das Potentiometer 6 die Winkelstellung des Orthesengelenks 4a. Die analogen Signale des Potentiometers 6 (Istwerte) werden in dem Analog-Digital-Wandler des Steuerelements 82 in digitale Signale umgewandelt und mit im Speicherelement des Bedienelements gespeicherten Referenzwerten (Sollwerten) verglichen. Je nachdem, ob die durch Referenzwerte vorgegebene Position des Orthesengelenks 4a erreicht ist oder nicht, stimmen die Referenzwerte mit den Istwerten überein oder nicht. Stimmen die Werte nicht überein gibt der Mikroprozessor im Bedienelement 80 das Signal an das Steuerelement 82 zum Fortsetzen der Bewegung. Das Steuerelement gibt das Signal an den Motor 62 der Antriebseinheit 5a weiter.

Die Referenzwerte können beispielsweise über den manuellen Teach-In-Modus der Antriebseinheit 5a in der Steuereinheit einprogrammiert werden. Dazu wird die Antriebseinheit 5a durch Öffnen des Kupplungshebels 55 (vgl. Figur 11 a) bis j)) aus dem Orthesengelenk 4a ausgekuppelt und somit in den Teach-In-Modus versetzt. Der Motor 62 und die Abtriebswelle 54 sind in diesem Zustand voneinander entkoppelt. Wird nun das Orthesengelenk 4a durch Muskelkraft bewegt, dreht sich die Abtriebswelle 54 um ihre Drehachse, wobei der Drehwinkel der Abtriebswelle 54 und damit die Winkelstellung des Orthesengelenks 54 vom Potentiometer 6, welches mit der Abtriebswelle 6 verbunden ist, gemessen wird. Zur Aufnahme von Referenzwerten für Extremstellungen des Sprunggelenks des Patienten, welche die Anschlagswerte für das Orthesengelenk 4a darstellen, wird das Sprunggelenk des Patienten entweder durch die eigene Muskelkraft oder durch den Therapeuten in eine Extremstellung bewegt und über das Bedienelement 80 ein Teach-In-Befehl ausgelöst. Die Winkelstellung des Orthesengelenks 4a bzw. der zugehörige Wert des Potentiometers 6 in dieser Extremstellung wird dann im Speicherelement des Bedienelements 80 gespeichert. Auf die gleiche Weise können Winkelstellungen für weitere Extremstellungen des Sprunggelenks gespeichert werden.

## Patentansprüche

1. Orthese mit mindestens einem ersten Halteteil und einem zweiten Halteteil, mindestens einem Orthesengelenk, welches das erste Halteteil mit dem zweiten Halteteil beweglich zueinander verbindet, und mindestens einem Gelenkantrieb zum Bewegen des Orthesengelenks, wobei das erste Halteteil geeignet ist zum Befestigen der Orthese an einem ersten Körperteil und das zweite Halteteil geeignet ist zum Führen eines mit dem ersten Körperteil durch ein Gelenk verbundenen zweiten Körperteils entlang vorbestimmter Freiheitsgrade bezüglich des ersten Körperteils,
**dadurch gekennzeichnet, dass**
die Orthese Befestigungselemente aufweist, mittels derer der Gelenkantrieb jederzeit zur muskulär und/oder Eigenkraft geführten Bewegung der Halteteile zueinander von dem Orthesengelenk entkoppelbar ist und zur elektronisch gesteuerten Bewegung der Halteteile zueinander an das Orthesengelenk ankoppelbar ist.

2. Orthese nach dem vorhergehenden Anspruch, **dadurch gekennzeichnet, dass** der Gelenkantrieb jederzeit zur muskulär und/oder Eigenkraft geführten Bewegung der Halteteile zueinander von der Orthese abnehmbar und zur elektronisch gesteuerten Bewegung der Halteteile zueinander an die Orthese anbringbar ist.

3. Orthese nach einem der vorhergehenden Ansprüche, **dadurch gekennzeichnet, dass** zur elektronisch gesteuerten Bewegung der Halteteile zueinander der Gelenkantrieb anstelle des Orthesengelenks anbringbar ist.

4. Orthese nach Anspruch 1, **dadurch gekennzeichnet, dass** der Gelenkantrieb eine Kupplung aufweist, wodurch der Gelenkantrieb vom Orthesengelenk auskuppelbar bzw. in das Orthesengelenk einkuppelbar ist.

5. Anordnung zur therapeutischen Beübung eines ersten und eines zweiten Körperteils und/oder eines die Körperteile verbindenden Gelenks mit
einer Orthese nach einem der vorhergehenden Ansprüche,
einem Sensor zum Messen und/oder Überwachen einer Stellung und/oder einer Drehgeschwindigkeit des Orthesengelenks zu verschiedenen Zeitpunkten, zum Messen einer Mittelstellung, Endstellung, eines Beschleunigungsprofils und/oder eines Drehmoments des Orthesengelenks,
einer Steuereinheit, welche
ein Speicherelement zum Speichern von Therapiedaten, insbesondere von Messwerten des Sensors während eines Trainingsablaufs, eines Steuerungsablaufs während eines Trainingsablaufs und/oder von Bewegungen während eines Trainingsablaufs,
einen Mikroprozessor und ein Bedienelement zum Programmieren und Ausführen eines Bewegungsprogramms und
ein Steuerelement zum Steuern des Gelenkantriebs aufweist.

6. Anordnung nach dem vorhergehenden Anspruch, **dadurch gekennzeichnet, dass** die Steuereinheit zum Herstellen einer Verbindung zu einem drahtlosen Netzwerk, z.B. lokales Drahtlosnetzwerk, Mobilfunknetz, Bluetoothverbindung, einen Netzwerkanschluss, z.B. WLAN-Netzwerkkarte, Mobilfunk-Schnittstelle, Bluetooth-Sender/Empfänger, aufweist, wodurch in der Speichereinheit speicherbare Daten über das drahtlose Netzwerk mittels eines Mikroprozessor-gesteuerten Geräts oder/und mittels eines tragbaren Mikroprozessor-gesteuerten Geräts, z.B. Netbook, Laptop, Handy, Smartphone, abrufbar und/oder auslesbar sind und/oder wodurch eine Therapie dokumentierbar, eine Qualitätssicherung und/oder eine Wartung der Orthese durchführbar und/oder die Therapie änderbar ist.

7. Anordnung nach Anspruch 5, **dadurch gekennzeichnet, dass** die Steuereinheit zum Herstellen einer Verbindung zu einem drahtgebundenen Netzwerk, z.B. lokales Netzwerk, Internet, einen Netzwerkanschluss, z.B. LAN-Netzwerkkarte, aufweist, wodurch in der Speichereinheit speicherbare Daten über das drahtgebundene Netzwerk mittels eines Mikroprozessor-gesteuerten Geräts oder/und mittels eines tragbaren Mikroprozessor-gesteuerten Geräts, z.B. Netbook, Laptop, abrufbar und/oder auslesbar sind und/oder wodurch eine Therapie dokumentierbar, eine Qualitätssicherung und/oder eine Wartung der Orthese durchführbar und/oder die Therapie änderbar ist.

8. Verfahren zur therapeutischen Beübung eines Gelenks und/oder eines ersten Körperteils und eines mit dem ersten Körperteil durch ein Gelenk verbundenen zweiten Körperteils mit einer Orthese nach einem der Ansprüche 1 bis 4 oder mit einer Anordnung nach einem der Ansprüche 5 bis 7, wobei die Orthese mittels eines ersten Halteteils am ersten Körperteil befestigt wird, und das zweite Körperteil mittels des zweiten Halteteils entlang vorbestimmter Freiheitsgrade geführt wird.

9. Verfahren nach Anspruch 8, **dadurch gekennzeichnet, dass** mittels des Bedienelements und des Mikroprozessors eine Bewegungsvorlage programmiert wird.

10. Verwendung einer Orthese nach einem der Ansprüche 1 bis 4, einer Anordnung nach einem der Ansprüche 5 bis 7 und/oder eines Verfahrens nach einem der Ansprüche 8 und 9 zur therapeutischen Beübung eines Gelenks und/oder zur Korrektur von Fehlstellungen eines ersten Körperteils und eines mit dem ersten Körperteil durch ein Gelenk verbundenen zweiten Körperteils und/oder eines die Körperteile verbindenden Gelenks.
